(19) **Europäisches Patentamt**
**European Patent Office**
**Office européen des brevets**

(11) **EP 3 349 728 B1**

(12) **EUROPEAN PATENT SPECIFICATION**

(45) Date of publication and mention
of the grant of the patent:
**26.02.2025 Bulletin 2025/09**

(21) Application number: **16767244.3**

(22) Date of filing: **16.09.2016**

(51) International Patent Classification (IPC):
*A61K 9/08* (2006.01)    *A61K 47/10* (2017.01)
*A61K 38/24* (2006.01)    *A61K 47/18* (2017.01)
*A61P 15/08* (2006.01)

(52) Cooperative Patent Classification (CPC):
**A61K 38/24; A61K 9/0019; A61K 47/10;
A61K 47/183; A61P 15/08**

(86) International application number:
**PCT/EP2016/071917**

(87) International publication number:
**WO 2017/046295 (23.03.2017 Gazette 2017/12)**

(54) **MAMMALIAN FOLLICLE-STIMULATING HORMONE COMPOSITION WITH INCREASED STABILITY**

ZUSAMMENSETZUNG AUS DEM FOLLIKELSTIMULIERENDEN HORMON VON SÄUGERN MIT ERHÖHTER STABILITÄT

COMPOSITION D'HORMONE DE STIMULATION FOLLICULAIRE MAMMIFÈRE À STABILITÉ ACCRUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.09.2015 LU 92831**

(43) Date of publication of application:
**25.07.2018 Bulletin 2018/30**

(73) Proprietor: **Guangzhou Canton Biologics Co., Ltd.
Huangpu District
510705 Guangzhou (CN)**

(72) Inventors:
• **GOLETZ, Steffen
13125 Berlin (DE)**

• **STOECKL, Lars
13125 Berlin (DE)**

(74) Representative: **Schiweck Weinzierl Koch
Patentanwälte Partnerschaft mbB
Ganghoferstraße 68 B
80339 München (DE)**

(56) References cited:
**EP-A2- 0 974 359    WO-A2-2011/099036**

Remarks:
The complete document including Reference Table(s) and the Sequence Listing(s) can be downloaded from the EPO website

## Description

## BACKGROUND

In general, the present disclosure pertains to the field of gonadotropins. In particular, an improved composition comprising recombinant human follicle-stimulating hormone (FSH), wherein the recombinant FSH has a human glycosylation pattern, and chlorocresol is provided. This improved composition has an increased stability at high temperatures and is useful in the treatment of infertility, in particular in human patients.

Gonadotropins are a group of protein hormones, which regulate gonadal function in the male and female and thereby play an important role in human fertility. They are secreted by gonadotrope cells of the pituitary gland of vertebrates after stimulation by the gonadotropinreleasing hormone (GnRH). Gonadotropins are heterodimeric glycoproteins including follicle stimulating hormone (FSH), luteinizing hormone (LH) and chorionic gonadotropin (CG). The gonadotropins share identical alpha-subunits but comprise different beta-subunits, which ensure receptor binding specificity.

FSH comprises a 92 amino acid alpha-subunit and a 111 amino acid beta-subunit, which confers specific binding to the FSH receptor. Both subunits of the natural protein are modified by glycosylation. The alpha-subunit is naturally glycosylated at Asn52 and Asn78 and the beta-subunit at Asn7 and Asn24. Both subunits are produced in the cells as precursor proteins and then processed and secreted. FSH regulates the development, growth, pubertal maturation, and reproductive processes of the body. In particular, it stimulates the maturation of germ cells and thus is involved in spermatogenesis and folliculogenesis.

Folliculogenesis is induced by FSH, for example, by binding of FSH to FSH receptors on the surface of granulosa cells. FSH receptors are G protein-coupled receptors, which activate the coupled G protein upon binding of FSH. The G protein in turn activates adenylyl cyclase, resulting in the production of cAMP, a second messenger molecule. The increasing cAMP concentration in the cell activates several downstream targets, in particular cAMP dependent protein kinases, which then lead to the synthesis of progesterone and estradiol. The progesterone and estradiol is secreted by the granulosa cells, inducing folliculogenesis. Upon stimulation of the granulosa cells by FSH, they also release inhibin-B, which forms a negative feedback loop, inhibiting the production and secretion of FSH in the pituitary gland. Inhibin-B was shown to be a good surrogate marker for the ovarian stimulation by FSH.

FSH is widely used in the treatment of infertility, either alone or in combination with other agents, in particular LH. In the art, generally FSH purified from post-menopausal human urine (urinary FSH) or FSH recombinantly produced by Chinese hamster ovary (CHO) cells has been used for human treatment. However, there is considerable heterogeneity associated with FSH preparations due to different isoforms present. Individual FSH isoforms exhibit identical amino acid sequences but differ in the extent and nature of their glycosylation. Particular isoforms are characterized by heterogeneity of the carbohydrate branch structures and differing amounts of sialic acid (a negatively charged terminal monosaccharide unit) incorporation, both of which influence the specific bioactivity of the isoform. Thus, the glycosylation pattern of the FSH has a significant influence on its biological activity.

However, urinary FSH from different donors and different preparations can significantly vary in its carbohydrate structures, resulting in a high batch-to-batch variation. There are also safety concerns regarding the presence of viruses in the urinary products. Furthermore, FSH obtained from CHO cells exhibits a glycosylation pattern specific for these hamster cells, which is not identical to human glycosylation patterns. These differences result in varying biological activities and adverse effects of the obtained FSH and thus, of the pharmaceutical preparations which are to be administered to the patient. Therefore, in the art recombinant FSH is preferably produced using suitable mammalian or human cell lines. Such recombinant FSH comprises an optimized homogeneous glycosylation pattern and thus a high bioactivity when administered to a patient.

In order to facilitate a convenient and safe administration stable and sterile FSH formulations are needed. In the art, preservatives are used to avoid microbial contamination in the formulation. However, such preservative agents are known to be potentially detrimental to the stability of proteins and therefore may have an adverse effect on the FSH preparation.

In the art different methods for infertility treatment using FSH exist. Some protocols require the daily administration of a FSH dose for up to 14 days and more to a patient. A stable FSH solution that can ideally be used for more than one time would omit the requirement to prepare daily FSH preparations for administration and would therefore be desirable.

Consequently, there is an unsatisfied need for making available and thus providing a FSH preparation, the FSH comprising an optimized homogeneous glycosylation pattern, wherein the preparation has preferably an increased stability.

The present invention satisfies this need by providing a stable FSH preparation that can also be used more than one time.

Surprisingly, the present inventors found that the preservative chlorocresol increases the stability of FSH peptides with human glycosylation patterns at higher temperatures. Given that, the group of preservatives, to which chlorocresol belongs, tends to be detrimental to protein stability, this finding was unexpected and is contrary to what is state of the art.

***

It must be noted that as used herein, the singular forms "a", "an", and "the", include plural references unless the context clearly indicates otherwise. Thus, for example, reference to "an expression cassette" includes one or more of the expression cassettes disclosed herein.

Unless otherwise indicated, the term "at least" preceding a series of elements is to be understood to refer to every element in the series.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

The term "about" or "approximately" as used herein means within 20%, preferably within 10%, and more preferably within 5% of a given value or range. It includes also the concrete number, e.g., about 20 includes 20.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The methods and techniques of the present invention are generally performed according to conventional methods well-known in the art. Generally, nomenclatures used in connection with techniques of biochemistry, enzymology, molecular and cellular biology, microbiology, genetics and protein and nucleic acid chemistry and hybridization described herein are those well-known and commonly used in the art.

The methods and techniques of the present invention are generally performed according to conventional methods well-known in the art and as described in various general and more specific references that are cited and discussed throughout the present specification unless otherwise indicated. See, e. g., Sambrook et al., Molecular Cloning: A Laboratory Manual, 3rd ed., Cold Spring Harbor Laboratory Press, Cold Spring Harbor, N. Y. (2001); Ausubel et al., Current Protocols in Molecular Biology, J, Greene Publishing Associates (1992, and Supplements to 2002); Handbook of Biochemistry: Section A Proteins, Vol I 1976 CRC Press; Handbook of Biochemistry: Section A Proteins, Vol II 1976 CRC Press.

The teaching disclosed in the PCT patent application no. WO 2012/017058 applies mutatis mutandis to the present invention.

## Summary

The present inventors have found that a composition comprising a recombinant FSH having a human glycosylation pattern and the preservative chlorocresol has advantageous properties with respect to the stability of the recombinant FSH. Accordingly, the present invention relates to a composition comprising recombinant FSH and chlorocresol, wherein the recombinant FSH has a human glycosylation pattern. The present invention also relates to said composition, wherein the recombinant FSH has an increased stability at high temperatures compared to a recombinant FSH with a mammalian glycosylation pattern comprised in a composition without chlorocresol, wherein the high temperature is preferably 37°C or more. The present invention also relates to said composition as defined herein further comprising a surfactant (e.g. Poloxamer 188), a tonicity modifier, a buffering agent, a stabilizer (e.g. L-methionine) and/or an excipient, wherein the surfactant is preferably Poloxamer 188 and wherein the stabilizer is preferably L-methionine. The present invention also relates to said composition as defined herein, wherein the recombinant FSH in the composition has a glycosylation pattern comprising one or more of the following characteristics: (i) a relative amount of glycans carrying bisecting N-acetylglucosamine (bisGlcNAc) of at least 20%; (ii) a relative amount of glycans carrying fucose of at least 30%; (iii) a relative amount of 2,6-coupled sialic acid of at least 30%; or (iv) it is a diverse glycosylation pattern, wherein the glycosylation pattern preferably comprises at least two of the features (i), (ii) and (iii), and more preferably all of the features (i), (ii) and (iii). The present invention also relates to said composition as defined herein, wherein the recombinant FSH is obtainable by production (i) in the human cell line GT-5s or a cell line derived therefrom or a cell line homologous thereto; or (ii) in the human cell line PerC6. The present invention also relates to said composition as defined herein, wherein the recombinant FSH in the composition comprises one or more of the following characteristics: (a) the glycosylation pattern comprises a relative amount of glycans carrying one or more sialic acid residues of at least 85%; (b) the glycosylation pattern comprises a relative amount of at least tetraantennary glycans of at least 18%; (c) a Z-number of at least 200; (d) it is human recombinant FSH; or (e) it is produced by a human cell line or human cells. In addition, the present invention also relates to said composition as defined herein, wherein the recombinant FSH in the composition has a glycosylation pattern comprising one or more of the following characteristics: (i) a relative amount of glycans carrying bisecting N-acetylglucosamine (bisGlcNAc) in the range of from 25% to 50%; (ii) a relative amount of at least tetraantennary glycans of at least 16%; (iii) a relative amount of glycans carrying fucose of at least 35%; (iv) a relative amount of 2,6-coupled sialic acid of at least 53%; (v) a relative amount of glycans carrying one or more sialic acid residues of at least 88%; (vi) a Z-number of at least 220; (vii) a relative amount of glycans carrying galactose of at least 95%; (viii) a relative amount of glycan branches carrying a terminal galactose unit optionally modified by a sialic acid residue of at least 60%; (ix) a relative amount of glycans carrying a sulfate group of at least 3%; (x) it comprises at least 45 different glycan structures, wherein each one of the different glycan structures has a relative amount of at least 0.05% of the total amount of glycan structures of the FSH in the composition; (xi) it comprises at least 35 different glycan structures, wherein each one of the different glycan structures has a relative amount of at least 0.1% of the total amount of glycan structures of the FSH in the composition; (xii) it comprises at least 20 different glycan structures, wherein each one of the different glycan structures has a relative amount of at least 0.5% of the total amount of glycan structures of the FSH in the composition; or (xiii) it comprises at least 40% more different glycan structures than FSH obtained from CHO cells in a corresponding composition, wherein each one of the different glycan structures has a relative amount of at least 0.05% of the total amount of glycan structures of the FSH in the respective composition. Further, the present invention relates to said composition as defined herein, wherein the recombinant FSH in the composition has a glycosylation pattern comprising the following characteristics: (i) a relative amount of glycans carrying bisecting N-acetylglucosamine (bisGlcNAc) in the range of from 25% to 50%; (ii) a relative amount of at least tetraantennary glycans of at least 16%; (iii) a relative amount of glycans carrying fucose of at least 35%; (iv) a relative amount of 2,6-coupled sialic acid in the range of from 53% to 99%; and (v) a relative amount of glycans carrying one or more sialic acid residues of at least 88%. In addition, the present invention relates to said composition as defined herein, wherein the recombinant FSH in the composition is capable of stimulating the release of progesterone in granulosa cells (a) at concentrations where no significant amounts of cAMP are released; and/or (b) by inducing a signal transduction pathway which is independent of cAMP signaling; and/or wherein the recombinant FSH in the composition is capable of stimulating or co-stimulating germ cell maturation by a biological process which is independent of cAMP signaling. Further, the present invention relates to said composition as defined herein, wherein the recombinant FSH in the composition has one or more of the following characteristics as can be determined in a granulose cell assay (a) it is capable of stimulating the release of progesterone in granulose cells at concentrations which are below the minimum concentration needed for the induction of cAMP release by the granulose cells; (b) it is capable of stimulating the release of at least 200 ng/ml progesterone in $5*10^4$ to $1*10^5$ granulosa cells/ml at FSH concentrations which do not induce a cAMP release or which induce a cAMP release of less than 10 pmol/ml; (c) it is capable of stimulating the release of at least 100 ng/ml progesterone in $5*10^4$ to $1*10^5$ granulosa cells/ml at a concentration that is lower than the concentration needed by human urinary FSH or recombinant FSH produced in CHO cells (Gonal F); and/or (d) it is capable of stimulating the release of at least 100 ng/ml progesterone in $5*10^4$ to $1*10^5$ granulosa cells/ml at a concentration wherein human urinary FSH or recombinant FSH produced in CHO cells (Gonal F) do not result in a corresponding release of progesterone. The present invention also relates to said composition as defined herein, wherein the recombinant FSH in the composition is capable of inducing follicle growth in a female human being after administration of a single dose, wherein the single dose preferably comprises 25 to 500 IU FSH and preferably is administered parenteral, in particular by subcutaneous injection. Additionally, the present invention relates to said composition as defined herein, wherein the recombinant FSH is FSH-GEX or wherein the composition is a pharmaceutical composition.

The present invention also relates to said composition as defined herein or to said pharmaceutical composition for use in infertility treatment. The present invention also relates to said composition as defined herein or to said pharmaceutical composition as defined herein for the use in infertility treatment, wherein (i) the dose to be administered to the patient results in an FSH concentration in the circulation of the patient in the range of 0.2 to 10 IU/L, preferably 0.4 to 7 IU/L; (ii) infertility treatment comprises inducing and/or stimulating the secretion of sex steroids independent of cAMP; (iii) infertility treatment comprises stimulating or co-stimulating germ cell maturation by a biological process independent of cAMP signaling; (iv) infertility treatment comprises inducing and/or stimulating the secretion of sex steroids at FSH concentrations at which no significant cAMP release is induced; and/or (v) wherein the infertility treatment includes assisted reproductive technologies, ovulation induction, in-vitro fertilization, for example in-vitro fertilization with intracytoplasmic sperm injection, gamete intrafallopian transfer, intrauterine insemination, treatment of anovulatory disorder in women, treatment of severe hormone deficiency disorder for egg maturation in woman, treatment of sperm production deficiencies in men, and/or the enablement or improvement of germ cell maturation such as folliculogenesis and spermatogenesis, in particular follicle maturation in women, for example during in vitro fertilization stimulation protocols and/or for anovulatory disorder treatment. Finally the present invention also relates to said composition as defined herein for the use in infertility treatment, wherein the infertility treatment involves the induction of follicle growth and/or ovular maturation.

**FIGURE LEGENDS**

**Figure 1** Potency of FSH-GEX determined by in vitro HEK assay relative to the formulation without chlorocresol over the period of 24 month

**Figure 2** Dose response curve of FSH after 12 month storage at 4°C and 37°C either in presence of chlorocresol or without chlorocresol as control.

**Figure 3** Dynamic Light Scattering analysis (Z-Average mean) of FSH-GEX formulated with chlorocresol (CC), benzalkoniumchlorid (BAK) or without preservative as control (control (FB)).

**DETAILED DESCRIPTION**

The present inventors have found that a composition comprising a recombinant FSH having a human glycosylation pattern and the preservative chlorocresol has advantageous properties with respect to the stability of the recombinant FSH. Accordingly, the present invention relates to a composition comprising recombinant FSH and chlorocresol, wherein the recombinant FSH has a human glycosylation pattern.

A "FSH composition" may be any composition or substance comprising or consisting of FSH. It may be in solid or fluid form and may comprise further ingredients in addition to FSH. In particular, a FSH preparation may be a solution comprising FSH and a suitable solvent such as water and/or alcohol, or a powder obtained, for example, after lyophilisation of a solution containing FSH. Suitable examples of a FSH preparation are composition obtained after expression of FSH in cells, in particular after purification of the FSH, or pharmaceutical compositions comprising FSH. A FSH preparation may contain, in addition to FSH, for example solvents, diluents, excipients, stabilizers, preservatives, salts, adjuvants and/or surfactants as described herein. The terms "FSH preparation" is used herein in particular in the meaning of a "composition comprising FSH". These terms are preferably used synonymously herein.

The term "FSH" refers to follicle-stimulating hormone, a gonadotropin, FSH is a glycoprotein comprised of two subunits, labelled alpha and beta subunits. Preferably, the FSH is human FSH, in particular human FSH composed of an alpha subunit having the amino acid sequence of SEQ ID NO: 1 and an beta subunit having the amino acid sequence of SEQ ID NO: 2. However, one or more, such as 1, 1 or 2, up to 3, up to 5, up to 10 or up to 20, amino acid substitution, addition and/or deletions may be present in one or both subunits. Preferably, the amino acid sequence of the alpha subunit shares an overall homology or identity of at least 80%, more preferably at least 85%, at least 90%, at least 95% or at least 98% with the amino acid sequence according to SEQ ID NO: 1. Furthermore, the amino acid sequence of the beta subunit preferably shares an overall homology or identity of at least 80%, more preferably at least 85%, at least 90%, at least 95% or at least 98% with the amino acid sequence according to SEQ ID NO: 2. The subunits of the FSH are preferably two separate polypeptide chains, however, the term "FSH" as used herein also encompasses embodiments wherein the two subunits are covalently attached to each other, e.g. by cross-linking agents or a linking polypeptide chain, and embodiments, wherein one or both subunits are further divided into several polypeptide chains. Preferably, the FSH according to the invention is capable of binding to and/or activating the FSH receptor, preferably the human FSH receptor. The term "FSH" as used herein in particular refers to all FSH proteins in a preparation. Thus, the term "FSH" in particular refers to the entirety of all FSH proteins in a FSH preparation or composition.

Preferably, both subunits of the FSH protein comprise one or more carbohydrate structures attached to the polypeptide chain. More preferably, the carbohydrate structures are attached to asparagine residues of the subunits. In particularly preferred embodiments, the alpha subunit comprises two carbohydrate structures attached to Asn52 and Asn78 and/or the beta-subunit comprises two carbohydrate structures attached to Asn7 and Asn24. The amino acid residues carrying the carbohydrate structures are designated with respect to the human amino acid sequences of the alpha and beta subunits according to SEQ ID NOs: 1 and 2, respectively. The sugar part of human FSH is preferably composed of fucose, galactose, mannose, galactosamine, glucosamine, and/or sialic acid.

FSH as used according to the present invention is recombinant FSH, more preferably recombinant human FSH. The term "recombinant FSH" refers to FSH which is not naturally produced by a living human or animal body and then obtained from a sample derived therefrom, such as urine, blood or other body liquid, feces or tissue of the human or animal body. Preferably, recombinant FSH is obtained from cells, which have been biotechnologically, engineered, in particular cells, which have been transformed, or transfected with a nucleic acid encoding FSH or the alpha or beta subunits of FSH. According to preferred embodiments, recombinant FSH is obtained from human cells comprising an exogenous nucleic acid encoding FSH. Respective exogenous nucleic acids can be introduced e.g. by using one or more expression vectors, which can be introduced into the host ceil e.g. via transfection. Respective methods for recombinantly producing proteins and FSH are well known in the prior art and thus, need no further description.

The term "nucleic acid" includes single-stranded and double-stranded nucleic acids and ribonucleic acids as well as deoxyribonucleic acids. It may comprise naturally occurring as well as synthetic nucleotides and can be naturally or synthetically modified, for example by methylation, 5'- and/or 3'-capping.

The term "vector" is used herein in its most general meaning and comprises any intermediary vehicle for a nucleic acid which enables said nucleic acid, for example, to be introduced into prokaryotic and/or eukaryotic host cells and, where appropriate, to be integrated into a genome of the host cell. Vectors of this kind are preferably replicated and/or expressed in the host cells. A vector preferably comprises one or more selection markers for selecting host cells comprising the vector. Suitable selection markers are resistance genes which provide the host cell with a resistance e.g. against a specific antibiotic. Further suitable selection markers are, for example, genes for enzymes such as DHFR or GS. Vectors enabling the expression of recombinant proteins including FSH as well as suitable expression cassettes and expression elements which enable the expression of a recombinant protein with high yield in a host cell are well known in the prior art and are also commercially available, and thus, need no detailed description here.

The FSH as defined herein is glycosylated, i.e. it is modified by one or more, preferably four, oligosaccharides attached to the polypeptides chains. Specifically, the recombinant FSH according to the present invention has a human glycosylation pattern. These oligosaccharides, also named glycans or carbohydrates, may be linear or branched saccharide chains and preferably are complex-type N-linked oligosaccharide chains. Depending on the number of branches, the oligosaccharide is termed mono-, bi-, tri- or tetraantennary (or even pentaantennary). A monoantennary oligosaccharide is unbranched while a bi-, tri- or tetraantennary oligosaccharide has one, two or three branches, respectively. A glycoprotein with a higher antennarity thus has more oligosaccharide endpoints and can carry more functional terminal saccharide units such as, for example, sialic acids. "At least triantennary" as used herein refers to oligosaccharides having an antennarity of at least 3, including triantennary, tetraantennary and pentaantennary oligosaccharides. "At least tetraantennary" as used herein refers to oligosaccharides having an antennarity of at least 4, including tetraantennary and pentaantennary oligosaccharides. With respect to complex-type N- glycans, a bisecting GlcNAc residue preferably is not considered as a branch or antenna and thus, does not add to the antennarity of the FSH.

The human glycosylation pattern of recombinant FSH as referred to herein in particular refers to the overall human glycosylation pattern of all FSH proteins in a FSH preparation according to the present invention. In particular, any glycan structures comprised in the FSH protein and thus, attached to the FSH polypeptide chains in the FSH preparation are considered and reflected in the human glycosylation pattern.

The composition of the invention further comprises the preservative chlorocresol. The term "preservative" refers to a compound or compositions added to a formulation to act as an antimicrobial, anti-fungal, anti-mycoplasmal, anti-viral, antiprion and/or anti-bacterial agent. Chlorocresol is preferably used in an amount that will yield a concentration that is effective to maintain the formulation essentially contamination free (suitable for injection). The preservative chlorocresol is preferably present in a concentration of at or about 0.005% (mass bacteriostatic/mass of solvent) to at or about 1.00%, more preferably at or about 0.02% (mass bacteriostatic/mass of solvent) to at or about 0.15%, most preferably at or about 0.05% to at or about 0.1%.

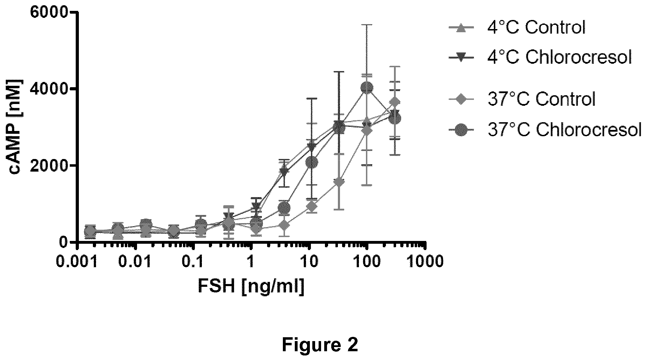

Surprisingly, the preservative chlorocresol used in the presently claimed composition does not adversely affect the biological activity of FSH by denaturing of the protein, e.g. by affecting the heterodimeric structure of FSH. Moreover the preservative chlorocresol surprisingly increases the stability of the recombinant FSH with a human glycosylation pattern at high temperatures compared to a recombinant FSH with a mammalian glycosylation pattern comprised in a composition without chlorocresol. This finding is in particular surprising because it was not known that preservatives increase the stability of FSH comprised in a composition when added to said composition. Furthermore, this effect pertains to the combination of the preservative chlorocresol and recombinant FSH with a human glycosylation pattern. The inventors did not observe an increased stability when using the preservative benzalkonium chloride in a composition comprising FSH (see Figure 2).

Said high temperature at which an increased stability may be observed is at least 25°C, at least 30°C, at least 35°C, 40°C, at least 45°C, at least 50°C, at least 55°C, at least 60°C, at least 65°C, at least 70°C, at least 75°C, at least 80°C, at least 85°C or at least 90°C. In a preferred embodiment, the high temperature is 37°C or more.

The term "stability" refers to the physical, chemical, and conformational stability of FSH in the composition of the present invention (including maintenance of biological potency). Instability of a protein formulation may be caused by chemical degradation or aggregation of the protein molecules to form higher order polymers, by dissociation of the heterodimers into monomers, deglycosylation, modification of glycosylation, oxidation (particularly of the a-subunit) or any other structural modification that reduces at least one biological activity of an FSH polypeptide included in the composition of the present invention. Due to the increased stability, the FSH composition of the present invention has an increased shelf-life.

The composition of the present invention may further comprise a surfactant to reduce aggregation. These additives are particularly useful if a pump or plastic container is used to administer the formulation by minimizing the loss of biological potency caused by adsorption on the surfaces of the vial and/or delivery device (e. g. syringe, pump, catheter, etc.). The presence of pharmaceutically acceptable surfactant mitigates the propensity for the protein to aggregate and thereby reduces the loss of biological potency. Suitable surfactants may be selected from block copolymers of ethylene oxide and propylene oxide, preferably Pluronic F77, Pluronic F87, Pluronic F88 and PluroniclD F68. In a preferred embodiment of the invention the surfactant added to the composition of the present invention is Poloxamer 188.

The composition of the present invention may further comprise a tonicity modifier. A "tonicity modifier" is a compound that is physiologically tolerated and imparts a suitable tonicity to a formulation to prevent the net flow of water across cell membranes that are in contact with the formulation. Compounds, such as glycerin, are commonly used for such purposes at known concentrations. Other suitable tonicity modifiers include, but are not limited to, amino acids or proteins (e. g., methionine or albumin), salts (e. g., sodium chloride), and sugars (e. g., dextrose, sucrose and lactose), and/or many others well known in the art.

The composition of the present invention may further comprise a buffering agent. The term "buffering agent" refers to solutions of compounds that are known to be safe for pharmaceutical or veterinary use in formulations and that have the effect of maintaining or controlling the pH of the formulation in the pH range desired for the formulation. Acceptable buffers for controlling pH at a moderately acidic pH to a moderately basic pH include, but are not limited to, such compounds as phosphate, acetate, citrate, arginine, TRIS, and histidine. "TRIS" refers to 2-amino-2-hydroxymethyl-1, 3,-propanediol, and to any pharmacologically acceptable salt thereof. Preferred buffers comprised by the composition of the invention include phosphate buffers, most preferably sodium phosphate, particularly phosphate buffered saline (PBS).

The composition may cover a wide range of pHs, such as from about pH 4 to about pH 10. Preferably compositions of FSH of the present invention have a pH between at or about 5.5 and at or about 9.0, more preferably at or about 6.0 to at or about 8.5, including about pH 7.0, pH 8. 0, and 8.2, most preferably at or about pH 7. 0.

The composition of the present invention may further comprise a stabilizer. The term "stabilizer" refers to a compound that is added to the composition of the present invention in order to minimize protein degradations including aggregation, inactivation, and oxidation. Such degradations may be accelerated by external factors such as heat and light, or in formulations that are free of human blood products such as albumin, or in multi-dose formulations which contain preservatives.

It is also envisioned herein that the stabilizer is L-methionine. US 5272135 describes that the addition of free L-methionine to the composition inhibits the oxidation of the methionine residues in polypeptides having amino acid sequences comprising at least one methionine residue. The oxidation of the methionine residues is said to be associated with the plastic containers, e.g., polypropylene or low density polyethylene (LDPE), which are readily permeable to oxygen, and within which the preparations are stored.

It is further envisioned herein that the composition may comprise an excipient. Examples of excipients are described herein and further examples are known by the skilled artisian.

Moreover, the recombinant FSH in the composition of the present invention preferably has an improved human glycosylation pattern. In particular, the high amount of bisecting GlcNAc residues and/or the high amount of 2,6-coupled sialic acids as well as the high amount of sulfated glycans may be responsible for the high activity of the FSH. Furthermore, the FSH compositions according to the present invention also show a much more diverse and complex glycosylation pattern, meaning that more different glycan structures are present in the preparation compared to conventional FSH preparations obtained e.g. from CHO cells. It is believed that the CHO-derived FSH is only able to activate one single pathway in the target cells while the improved FSH according to the present invention, due to its unique glycosylation pattern, apparently exerts its biological activity via multiple different pathways, resulting in an increased biological response. Some of these pathways involve cAMP signaling while other, novel pathways are cAMP independent.

Accordingly, the FSH in the composition preferably has a glycosylation pattern comprising one or more of the following characteristics:

(i) a relative amount of glycans carrying bisecting N-acetylglucosamine (bisGlcNAc) of at least 20 %; and/or
(ii) a relative amount of glycans carrying fucose of at least 30%; and/or
(iii) a relative amount of 2,6-coupled sialic acid of at least 30%; and/or
(iv) it is a diverse glycosylation pattern.

It is also envisioned herein that the glycosylation pattern comprises at least two of the features (i), (ii) and (iii), and preferably all of the features (i), (ii) and (iii).

The term "sialic acid" in particular refers to any N- or O-substituted derivatives of neuraminic acid. It may refer to both 5-N-acetylneuraminic acid and 5-N-glycolylneuraminic acid, but preferably only refers to 5-N-acetyineuraminic acid. The sialic acid, in particular the 5-N-acetylneuraminic acid preferably is attached to a carbohydrate chain via a 2,3- or 2,6-linkage. Preferably, in the FSH compositions described herein both 2,3- as well as 2,6-coupled sialic acids are present.

A "relative amount of glycans" according to the invention refers to a specific percentage or percentage range of the glycans attached to the FSH glycoproteins in a composition comprising FSH. In particular, the relative amount of glycans refers to a specific percentage or percentage range of all glycans comprised in the FSH proteins and thus, attached to the FSH polypeptide chains in a composition comprising FSH. 100 % of the glycans refers to all glycans attached to the FSH glycoproteins of the FSH preparation or in a composition comprising FSH, respectively. For example, a relative amount of glycans carrying bisecting GlcNAc of 60% refers to a FSH preparation wherein 60% of all glycans comprised in the FSH proteins and thus, attached to the FSH polypeptide chains in said FSH preparation comprise a bisecting GlcNAc residue while 40% of all glycans comprised in the FSH proteins and thus, attached to the FSH polypeptide chains in said FSH preparation do not comprise a bisecting GlcNAc residue.

The numbers given herein, in particular the relative amounts of a specific glycosylation property are preferably to be understood as approximate numbers. In particular, the numbers preferably may be up to 10% higher and/or lower, in particular up to 9%, 8%, 7%, 6%, 5%, 4%, 3%, 2% or 1 % higher and/or lower.

A "relative amount of 2,6-coupled sialic acid" refers to a specific percentage or percentage range of the total amount of sialic acids being 2,6-coupled sialic acids. A relative amount of 2,6-coupled sialic acid of 100% thus means that all sialic acids are 2,6-coupled sialic acids. For example, a relative amount of 2,6-coupled sialic acids of 60% refers to a FSH preparation wherein 60% of all sialic acids comprised in the FSH proteins and thus, attached to the oligosaccharide chains of the FSH proteins in said FSH preparation are attached via a 2,6-linkage while 40% of all sialic acids comprised in the FSH proteins and thus, attached to the oligosaccharide chains of the FSH proteins in said FSH preparation are not attached via a 2,6-linkage, but for example via a 2,3-linkage or a 2,8-linkage.

The term "diverse glycosylation pattern" in particular refers to the glycosylation pattern of the FSH proteins in a preparation or composition which glycosylation pattern comprises multiple different glycan structures. Different glycan structures are oligosaccharide structures, which differ in the presence/absence, amount and/or position of at least one monosaccharide unit and/or at least one chemical modification such as e.g. sulfate residues, acetyl residues or the like. A specific "different glycan structure" preferably is only considered in this respect if its relative amount is at least 0.02 %, more preferably at least 0.03 %, at least 0.05 %, at least 0.07 %, at least 0.1 %, at least 0.15 %, at least 0.2 %, at least 0.25 %, at least 0.3 % or at least 0.5 % of the total amount of glycan structures in the glycosylation pattern. A diverse glycosylation pattern in particular is a glycosylation pattern, which comprises at least 5 different glycan structures. Preferably, the diverse glycosylation pattern comprises at least 7, more preferably at least 10, at least 15, at least 20, at least 25, at least 30, at least 35, at least 40, at least 45, at least 50, at least 55 and most preferably at least 60 different glycan structures. A diverse glycosylation pattern in particular also refers to a glycosylation pattern of FSH in a preparation or composition which glycosylation pattern comprises more different glycan structures than FSH obtained from CHO cells in a respective preparation or composition, in particular, the glycosylation pattern comprises at least 10 %, preferably at least 20 %, at least 30 %, at least 40 %, at least 50 %, at least 80 %, at least 70 %, at least 80%, at least 90 %, and most preferably at least 100 % more different glycan structures than FSH obtained from CHO cells.

The degree of sialylation of FSH is normally expressed as Z-number. The Z-number indicates the relative negative charge of the glycan structures of a glycoprotein. The Z- number is calculated by the formula:

$$Z = A1\% * 1 + A2\% * 2 + A3\% * 3 + A4\% * 4$$

wherein A1% is the percentage of glycans with a charge of -1 , A2% is the percentage of glycans with a charge of -2, A3% is the percentage of glycans with a charge of -3, and A4% is the percentage of glycans with a charge of -4. These percentages are calculated with respect to all glycans attached to the FSH, including charged as well as uncharged glycans. The charge of the glycans may be provided by any charged monosaccharide units or substituents comprised in the glycan, in particular by sialic acid residues and/or sulfate groups and/or phosphate groups. Since the charge of the glycans of FSH is generally only determined by their sialic acid residues and FSH generally has four glycan structures, the Z-number is an indication for the amount of sialic acids on the FSH or the acidify of the FSH. However, when the FSH also comprises a significant amount of sulfated glycans, the Z-number is an indication for the combined amounts of sialic acids and sulfate groups.

In certain embodiments, the present invention provides a recombinant FSH preparation, wherein the recombinant FSH in the preparation has a human glycosylation pattern comprising one or more of the following characteristics:

(i) a relative amount of glycans carrying bisecting N-acetylglucosamine (bisGlcNAc) of at least 35 %;
(ii) a relative amount of glycans carrying fucose of at least 60%; and
(iii) a relative amount of 2,6-coupled sialic acid of at least 30%.

Said recombinant FSH, having a human glycosylation pattern, is obtained by recombinant production in a human host cell. Suitable human host cells, which provide a respective glycosylation pattern, are described subsequently.

Preferably, the human glycosylation pattern comprises at least two of the features (i), (ii) and (iii) (in particular features (i) and (ii), (i) and (iii), or (ii) and (iii)), and more preferably all of the features (i), (ii) and (iii). Furthermore, the glycosylation pattern may further comprise a relative amount of at least tetraantennary glycans of at least 15%, and/or a relative amount of glycans carrying one or more sialic acid residues of at least 80%, and/or a relative amount of glycans carrying galactose of at least 95%, and/or a relative amount of glycan branches carrying a terminal galactose unit of at least 60%, and/or a relative amount of glycans carrying a sulfate group of at least 1 %, preferably at least 2.5%. The terminal galactose unit may optionally further carry a sialic acid residue. The recombinant FSH in the composition preferably has a Z-number of at least 200.

The relative amount of glycans carrying bisGlcNAc is preferably at least 25%, at least 27%, at least 30%, at least 35%, at least 38% or at least 40%. More preferably, it is in the range of from about 25% to about 60%, in particular in the range of from about 35% to about 60% or in the range of from about 38% to about 50% or in the range of from about 40% to about 45%. According to one embodiment, it is about 42%. The relative amount of glycans carrying one or more sialic acid residues is preferably at least 83%, at least 85% or at least 88%, and more preferably in the range of from about 85% to about 98% or in the range of from about 88% to about 95%, most preferably about 90%. The Z-number is preferably at least 210, more preferably at least 215, at least 220, at least 230 or at least 240. A higher Z-number is for example obtainable by enriching the FSH preparation for acidic and/or negatively charged FSH proteins. Preferably, the relative amount of at least tetraantennary glycans is at least 16%, at least 17%, at least 18% or at least 19%, more preferably at least 20% or at least 21 %. The relative amount of at least triantennary glycans, in particular tri- and tetraantennary glycans, preferably is at least 25%, at least 30%, at least 35% or at least 40%, more preferably at least 45%, at least 50% or at least 55%. Preferably, the relative amount of glycans carrying fucose is at least 35%, at least 40%, at least 50%, at least 60% or at least 70%, more preferably at least 75% or at least 78%. It may be in the range of from about 70% to about 90%, in particular in the range of from about 75% to about 85%. Preferably, the relative amount of 2,6-coupled sialic acid is at least 40%, at least 45%, at least 50%, at least 53%, at least 55%, at least 60% or at least 65%, in particular in the range of about 40 % to about 99%, preferably about 40% to about 80%, about 50% to about 60% or about 53 % to about 70 %, Preferably, the ratio of 2,3-coupled sialic acid to 2,6-coupled sialic acid is in the range of from about 1 :10 to about 7:3, more preferably from about 1 :5 to about 3:2 or from about 1 :2 to about 1 :1 , most preferably from about 2:3 to about 1 :1 . In preferred embodiments, the relative amount of 2,6- coupled sialic acids exceeds that of 2,3-coupled sialic acids. The relative amount of glycans carrying galactose preferably is at least 97% and most preferably is about 98%. Preferably, the relative amount of glycan branches carrying a galactose unit optionally modified by a sialic acid residue is at least 65%, more preferably at least 70% or at least 73%. It is preferably in the range of from about 60% to about 95%, and more preferably in the range of from about 70% to about 80%. Preferably, the relative amount of glycans carrying a sulfate group (sulfated glycans) is at least 1 %, at least 1.5%, at least 2%, at least 2.5%, at least 3% or at least 5%, more preferably at least 7%, at least 10% or at least 12%. According to one embodiment, the relative amount of glycans carrying a sulfate group does not exceed 50%, preferably 40%, 35%, 30%, 25% or 20%.

In preferred embodiments, the recombinant FSH, having a human glycosylation pattern, in the preparation has a diverse glycosylation pattern wherein the FSH in the preparation comprises at least 45 or preferably at least 50 different glycan structures, wherein each one of the different glycan structures has a relative amount of at least 0.05 % of the total amount of glycan structures of the FSH in the preparation. According to one embodiment, the FSH in the preparation comprises at least 35 or preferably at least 40 different glycan structures, wherein each one of the different glycan structures has a relative amount of at least 0.1 % of the total amount of glycan structures of the FSH in the preparation; and/or the FSH in the preparation comprises at least 20 or preferably at least 25 different glycan structures, wherein each one of the different glycan structures has a relative amount of at least 0.5 % of the total amount of glycan structures of the FSH in the preparation, in a further embodiment, the FSH in the preparation comprises at least 40 %, preferably at least 50 % more different glycan structures than FSH obtained from CHO cells in a corresponding preparation, wherein each one of the different glycan structures has a relative amount of at least 0.05 %, 0.1 % or 0.5% of the total amount of glycan structures of the FSH in the respective preparation.

In preferred embodiments, the recombinant FSH preparation according to the invention does not comprise N-glycolyl neuraminic acids (NeuGc) or detectable amounts of NeuGc. Furthermore, the recombinant FSH preparation according to the invention preferably also does not comprise Galili epitopes (Galα1,3-Gal structures) or detectable amounts of the Galili epitope.

As mentioned above, the present invention specifically provides a recombinant FSH with a human glycosylation pattern. Due to these glycosylation properties, foreign immunogenic nonhuman structures which induce side effects are absent which means that unwanted side effects or disadvantages known to be caused by certain foreign sugar structures such as the immunogenic non-human sialic acids (NeuGc) or the Galili epitope (Gal-Gal structures), both known for rodent production systems, or other structures like immunogenic high-mannose structures as known from e.g. yeast systems are avoided.

Accordingly, it is envisioned that the recombinant FSH, having a human glycosylation pattern, in the composition comprises one or more of the following characteristics

(a) the glycosylation pattern comprises a relative amount of glycans carrying one or more sialic acid residues of at least 85%;
(b) the glycosylation pattern comprises a relative amount of at least tetraantennary glycans of at least 18%;
(c) a Z-number of at least 200;
(d) it is human recombinant FSH; and/or

(e) it is produced by a human cell line or human cells.

In a further preferred embodiment of the invention the recombinant FSH, having a human glycosylation pattern, in the composition has a glycosylation pattern comprising one or more of the following characteristics:

(i) a relative amount of glycans carrying bisecting N-acetylglucosamine (bisGlcNAc) in the range of from about 25% to about 50%;
(ii) a relative amount of at least tetraantennary glycans of at least 16%;
(iii) a relative amount of glycans carrying fucose of at least 35%;
(iv) a relative amount of 2,6-coupled sialic acid of at least 53%;
(v) a relative amount of glycans carrying one or more sialic acid residues of at least 88%;
(vi) a Z-number of at least 220;
(vii) a relative amount of glycans carrying galactose of at least 95%;
(viii) a relative amount of glycan branches carrying a terminal galactose unit optionally modified by a sialic acid residue of at least 60%;
(ix) a relative amount of glycans carrying a sulfate group of at least 3%;
(x) it comprises at least 45 different glycan structures, wherein each one of the different glycan structures has a relative amount of at least 0.05 % of the total amount of glycan structures of the FSH in the preparation;
(xi) it comprises at least 35 different glycan structures, wherein each one of the different glycan structures has a relative amount of at least 0.1 % of the total amount of glycan structures of the FSH in the preparation;
(xiii) it comprises at least 20 different glycan structures, wherein each one of the different glycan structures has a relative amount of at least 0.5 % of the total amount of glycan structures of the FSH in the preparation; and/or
(xiv) it comprises at least 40 % more different glycan structures than FSH obtained from CHO cells in a corresponding preparation, wherein each one of the different glycan structures has a relative amount of at least 0.05 % of the total amount of glycan structures of the FSH in the respective preparation.

It is also envisioned herein that the recombinant FSH, having a human glycosylation pattern, in the composition has a glycosylation pattern comprising the following characteristics:

(i) a relative amount of glycans carrying bisecting N-acetylglucosamine (bisGlcNAc) in the range of from about 25% to about 50%;
(ii) a relative amount of at least tetraantennary glycans of at least 16%;
(iii) a relative amount of glycans carrying fucose of at least 35%;
(iv) a relative amount of 2,6-coupled sialic acid in the range of from about 53% to about 99%; and
(v) a relative amount of glycans carrying one or more sialic acid residues of at least 88%.

In certain preferred embodiments, the recombinant FSH, having a human glycosylation pattern, in the composition according to the invention has one of the glycosylation patterns listed in the following Table 1 :

Table 1: Specific glycosylation parameters

| Embodiment | B | 2,6-S | Sulfate | S>0 | Z | tetra |
|---|---|---|---|---|---|---|
| 1 | ≥ 20 | ≥ 53 | ≥ 2.5 | | | |
| 2 | ≥ 20 | ≥ 53 | ≥ 2.5 | ≥ 80 | ≥ 200 | ≥ 15 |
| 3 | ≥ 20 | ≥ 53 | ≥ 2.5 | ≥ 85 | | |
| 4 | ≥ 20 | ≥ 53 | ≥ 2.5 | | ≥ 220 | |
| 5 | ≥ 20 | ≥ 53 | ≥ 2.5 | | | ≥ 17 |
| 6 | ≥ 20 | ≥ 53 | ≥ 2.5 | ≥ 85 | ≥ 220 | ≥ 17 |
| 7 | 20-50 | ≥ 53 | ≥ 2.5 | | | |
| 8 | ≥ 20 | 53-80 | ≥ 2.5 | | | |
| 9 | ≥ 20 | ≥ 53 | 2.5-30 | | | |
| 10 | ≥ 20 | ≥ 53 | ≥ 2.5 | ≥ 80 | 200-260 | ≥ 15 |
| 11 | ≥ 20 | ≥ 53 | ≥ 2.5 | ≥ 80 | ≥ 200 | 15-30 |
| 12 | 20-50 | 53-80 | 2.5-30 | 80-100 | 200-260 | 15-30 |

(continued)

| Embodiment | B | 2,6-S | Sulfate | S>0 | Z | tetra |
|---|---|---|---|---|---|---|
| 13 | ≥ 25 | ≥ 55 | ≥ 3 | | | |
| 14 | ≥ 30 | ≥ 55 | ≥ 3 | | | |
| 15 | ≥ 25 | ≥ 60 | ≥ 3 | | | |
| 16 | ≥ 25 | ≥ 55 | ≥ 10 | | | |
| 17 | ≥ 30 | ≥ 60 | ≥ 10 | | | |
| 18 | ≥ 25 | ≥ 55 | ≥ 3 | ≥ 80 | ≥ 200 | ≥ 15 |
| 19 | ≥ 25 | ≥ 55 | ≥ 3 | ≥ 85 | ≥ 220 | ≥ 17 |
| 20 | ≥ 30 | ≥ 60 | ≥ 10 | ≥ 85 | ≥ 220 | ≥ 17 |

shown are the relative amounts of glycans having the following property:
B: bisecting GlcNAc; 2,6-S: 2,6-coupled sialic acid; sulfate: sulfated glycans; S>0: at least one sialic acid; Z: Z number; tetra: at least tetraantennary glycans

In embodiments 1 to 12 listed in table 1, preferably the relative amount of bisecting GlcNAc is at least 25% instead of at least 20%; and/or the relative amount of 2,6- coupled sialic acids preferably is at least 55%, more preferably at least 60%, instead of at least 53%; and/or the relative amount of sulfated glycans preferably is at least 3%, more preferably at least 10%, instead of at least 2.5%. The glycosylation patterns listed in table 1 preferably are human glycosylation patterns and/or do not comprise NeuGc and the Galili epitope.

The FSH according to the invention is a recombinant FSH having a human glycosylation pattern, obtainable by production in a human cell, preferably a human cell line. The human cell line preferably is derived from human blood cells, in particular it is a myeloid ceil line, preferably a myeloid leukemia cell line. The cell line preferably is immortalized. In a preferred embodiment, the cell line for the production of the FSH according to the invention is the cell line GT-5s, deposited on July 28, 2010 under the accession number DS ACC3078 according to the requirements of the Budapest Treaty at the Deutsche Sammlung von Mikroorganismen und Zellkulturen (DSMZ), Inhoffenstraße 7B, 38124 Braunschweig (DE) by the Glycotope GmbH, Robert-Rossle-Str, 10, 13125 Berlin (DE), or a cell line derived therefrom, or a homologous cell line. GT-5s is an immortalized human myeloid leukemia cell line, which is capable of providing a specific glycosylation pattern as described herein. According to the present invention, the terms "GT-5s" and "GT-5s cell line" also include cells or cell lines derived from GT-5s. A cell line, which is derived from GT-5s, can be for example obtained by randomly or specifically selecting a single clone or a group of ceils from a GT-5s culture, optionally after treating the GT-5s cells in order to enhance their mutation rate, or by genetically altering a GT-5s cell line. The selected clone or group of cells may further be treated as described above and/or further rounds of selection may be performed. A cell line, which is homologous to GT-5s, in particular is an immortalized human myeloid cell line. Preferably, a cell line derived from or homologous to GT-5s is capable of providing FSH having a glycosylation pattern similar to that obtained from GT-5s. Preferably, FSH that is produced by a cell line derived from or homologous to GT-5s has one or more of the glycosylation characteristics as described herein, in particular a relative amount of glycans carrying bisecting N-acetylglucosamine (bisGlcNAc) of at least 20 %; and/or a relative amount of glycans carrying fucose of at least 30%; and/or a relative amount of 2,6-coupied sialic acid of at least 30%; and/or a diverse glycosylation pattern. The cell line derived from or homologous to GT-5s is capable of expressing FSH having a glycosylation pattern as is described as preferred herein, in particular a glycosylation pattern selected from Table 1. The similar glycosylation pattern of FSH that is produced by the cell line derived from or homologous to GT-5s preferably differs from the glycosylation pattern of FSH obtained from GT-5s by 20% or less, more preferably 15% or less, 10% or less or 5% or less, in particular in one or more, preferably all of the glycosylation properties selected from the group consisting of the relative amount of bisGlcNAc, the relative amount of sialylated glycans, the relative amount of sulfated glycans, the relative amount of 2,6-coupled sialic acids, the relative amount of fucose, the relative amount of tetraantennary glycans, the relative amount of glycan branches carrying galactose, and the Z number. Furthermore, the FSH according to the invention preferably is human FSH, having one or more specific glycosylation characteristics as disclosed herein, preferably a glycosylation pattern selected from Table 1. The cell line GT-5s as well as cell lines derived therefrom and cell lines homologous thereto are in particular advantageous since they provide a very stable and homogeneous protein production, in particular with respect to FSH protein. They have a very good batch-to- batch consistency, i.e. the produced proteins and their glycosylation pattern are similar when obtained from different production runs or when produced at different scales and/or with different culturing procedures. In particular, the diverse glycosylation pattern as described herein is highly reproducible in different production runs using these cell lines for expressing FSH.

The terms "cell" and "cells" and "cell line" used interchangeably, preferably refer to one or more mammalian cells, in particular human cells. The term includes progeny of a cell or cell population. Those skilled in the art will recognize that "cells" include progeny of a single cell, and the progeny can not necessarily be completely identical (in morphology or of total DNA complement) to the original parent cell due to natural, accidental, or deliberate mutation and/or change. "Cell" preferably refers to isolated cells and/or cultivated cells, which are not incorporated in a living human or animal body.

Accordingly, the present invention provides a recombinant FSH composition that is obtainable by production in a human host cell or a human cell line. Preferably, the recombinant FSH is obtainable from a human myeloid cell line, preferably an immortalized human myeloid leukemia cell line, in particular the cell line GT-5s or a cell line derived therefrom or a cell line homologous to GT-5s. It was found that an FSH produced in said cell line exhibits a glycosylation pattern as described above and in particular exhibits the advantageous therapeutic and pharmacological effects described herein. Thus, the present invention also pertains to a method for producing a recombinant FSH preparation by recombinantly expressing the FSH in a suitable cell line, in particular a cell line as described above, preferably the cell line GT-5s, a cell line derived from GT-5s or a cell line homologous to GT-5s. The recombinant FSH may further be expressed in other human cell lines like PerC6, HT-1080 or HEK293 or a cell line derived from PerC6, HT-1080 or HEK293 or a cell line homologous to PerC6, HT-1080 or HEK293. The recombinant FSH respectively produced can be isolated and optionally purified.

Thus, the recombinant FSH composition preferably is obtainable by a process comprising the steps of:

(i) cultivating a human host cell, preferably derived from the cell line GT-5s or a homologous cell line or derived from the cell lines like PerC6, HT-1080 or HEK293 or a homologous cell line, comprising nucleic acids coding for the FSH alpha and beta subunits under conditions suitable for expression of the FSH; and
(ii) isolating FSH.

The human host cells used for expression preferably are myeloid cells, in particular immortalized myeloid leukemia cells, and preferably are or are derived from the cell line GT-5s or is a cell line homologous thereto or the cell lines like PerC6, HT-1080 or HEK293or a cell line derived from the cell lines like PerC6, HT-1080 or HEK293or a cell line homologous thereto. The human host cells are cultured so that they express FSH. Suitable culture conditions are known to the skilled person.

The isolation of recombinant FSH, having a human glycosylation pattern, preferably comprises the further steps of:

(a) obtaining the culture supernatant where the FSH is secreted by the human cells, or lysing the human cells where the FSH is not secreted;
(b) isolating the FSH from the culture supernatant or cell lysate using chromatographic steps such as reversed phase chromatography, size exclusion chromatography and/or hydrophobic interaction chromatography; and
(c) optionally obtaining an acidic fraction of the FSH by removing basic FSH isoforms, preferably by using anion exchange chromatography including a washing step which removes basic FSH isoforms, such as a washing step at about pH 5.0 or about pH 4.5 or about pH 4.0.

A suitable purification process for the recombinant FSH is described, for example, in the U.S. patent application no. US 61/263,931, the European patent application no. EP 09 014 585.5 and the PCT patent application no. WO 2011/063943.

Preferably, the nucleic acid coding for the FSH alpha subunit and the nucleic acid coding for the FSH beta subunit are comprised in expression cassettes comprised in a suitable expression vector that allows the expression in a human host cell. The nucleic acid coding for the FSH alpha subunit and the nucleic acid coding for the FSH beta subunit may be comprised in the same vector, but preferably are comprised in separate vectors. Furthermore, they may also be expressed from one expression cassette using appropriate elements such as an IRES element. Preferably, the FSH is secreted by the human cells. In preferred embodiments, cultivation of the human cells is performed in a fermenter and/or under serum-free conditions.

The recombinant FSH composition obtainable by production in human host cells or a human cell line preferably exhibits the features described herein with respect to the recombinant FSH preparation according to the present invention, in particular, its glycosylation pattern comprises one or more of the characteristics described above, preferably at least one glycosylation pattern as described in Table 1 and/or in claims 12 to 17.

The recombinant FSH, having a human glycolysation pattern, according to the present invention is recombinant human FSH (rhFSH), obtainable by production in a human cell line, such as the cell lines GT-5s, PerC6, HT-10-80, or HEK293, which comprises one or more nucleic acids encoding the human FSH subunits and elements for expressing said one or more nucleic acids in the host cell. Preferably, the alpha subunit of the rhFSH has the amino acid sequence according to SEQ ID NO: 1 or an amino acid sequence having a homology or preferably identity to SEQ ID NO: 1 over its entire length of at least 80%, preferably at least 85%, at least 90%, at least 95% or at least 98%. In preferred embodiments, the alpha subunit of the rhFSH comprises asparagine residues at positions 52 and 78 and is glycosylated at amino acids Asn52 and Asn78. The beta subunit of the rhFSH preferably has the amino acid sequence according to SEQ ID NO: 2 or an amino acid sequence having a homology or preferably identity to SEQ ID NO: 2 over its entire length of at least 80%, preferably at least 85%, at least 90%, at least 95% or at least 98%. In preferred embodiments, the beta subunit of the rhFSH comprises asparagine residues at positions 7 and 24 and is glycosylated at amino acids Asn7 and Asn24.

In a further preferred embodiment of the invention, the recombinant FSH composition according to the present invention is capable of stimulating the release of progesterone in granulosa cells

(a) at concentrations where no significant amounts of cAMP are released; and/or
(b) by inducing a signal transduction pathway which is independent of cAMP signaling.

According to the invention, the term "wherein no significant amounts of cAMP are released" or similar expressions, respectively, in particular refer to the release of cAMP by cells or tissue in an amount which is less than 25%, preferably less than 20%, more preferably less than 15%, less than 10%, less than 7.5%, less than 5% or less than 2.5% of the amount of cAMP release obtained by cells or tissue after stimulation with FSH in a concentration which results in the maximum release of cAMP. These cells or tissues are susceptible or responsive to stimulation by FSH, such as granulosa cells or Sertoli cells. A cAMP release, which is independent of FSH, i.e. a cAMP release that also occurs in the absence of FSH, should not be considered in this respect.

Preferably, a "release of a significant amount of cAMP" or a "significant release of cAMP" is any release of cAMP above the cAMP release in the absence of FSH, in particular any detectable release of cAMP above the inaccuracy of measurement. A standard procedure for measuring cAMP release is described in the examples and may be used for determining a significant or non-significant release of cAMP. The "release of cAMP" refers to an intracellular release of cAMP and/or an extracellular release or secretion of cAMP, preferably only to a secretion of cAMP. cAMP refers to cyclic adenosine monophosphate which acts as a second messenger molecule in cellular signal transduction. cAMP is synthesized in cells from ATP by the adenylyl cyclase. A biological process or signal transduction pathway which is "independent of cAMP signaling" preferably does not involve activation of adenylyl cyclase.

According to a further embodiment, the recombinant FSH preparation according to the present invention is capable of stimulating or co-stimulating germ cell maturation by a biological process, which is independent of cAMP signaling. It was surprisingly found in experiments that the glycosylation pattern described above results in a respective novel pharmacological profile of the recombinant FSH, which exhibits the pharmacological and therapeutic advantages described herein.

The recombinant FSH composition is according to one embodiment capable of stimulating the release of progesterone in granulose cells at concentrations which are below the minimum concentration needed for the induction of cAMP release by the granulose cells. The release of progesterone, estradiol and/or cAMP mentioned below refers to an in vitro release in about $1*10^4$ to about $1*10^6$ granulosa cells/ml, preferably in about $5*10^4$ to about $1*10^5$ granulosa cells/ml, in particular under conditions as described in example 2, below.

Preferably, the recombinant FSH preparation according to the present invention is capable of releasing at least 100ng/ml, at least 150 ng/ml, at least 200ng/ml, preferably at least 250ng/ml, at least 300 ng/ml or at least 400 ng/ml progesterone at a concentration which does not induce a cAMP release or which induces a cAMP release of less than 20 pmol/ml, less than 15 pmol/ml, less than 10 pmol/ml, less than 5pmol/ml.

Furthermore, the recombinant FSH composition according to the present invention is preferably capable of releasing at least 100ng/ml, at least 200ng/ml, preferably at least 300ng/ml or at least 400ng/ml progesterone at a FSH concentration that is lower than the concentration necessary with human urinary FSH or recombinant FSH produced in CHO cells (Gonal F), Thus, it is preferably capable of releasing at least 100ng/ml, at least 200ng/ml, preferably at least 300ng/ml or at least 400ng/ml progesterone at a concentration wherein human urinary FSH or recombinant FSH produced in CHO cells (Gonal F) do not result in a corresponding, respectively equally high release of progesterone. As is demonstrated by the examples, the recombinant FSH according to the present invention induces respectively stimulates the production of progesterone more strongly than human urinary FSH or recombinant FSH produced in CHO cells (Gonal F).

The recombinant FSH composition according to the present invention may have one or more of the subsequently described characteristics as can be determined in a granulose cell assay as described in the PCT patent application no. WO 2012/017058.

Furthermore, the recombinant FSH composition according to the present invention is preferably capable of releasing at least 50 nmol/l, at least 75 nmol/l, at least 100 nmol/l, at least 125 nmol/l or at least at least 150 nmol/l estradiol at a FSH concentration which does not induce a cAMP release or which induces a cAMP release of less than 20 pmol/ml, less than 15 pmol/ml, less than 10 pmol/ml, less than 5pmol/ml.

Furthermore, the recombinant FSH composition according to the present invention is preferably capable of releasing at least 50 nmol/l, at least 75 nmol/l, at least 100 nmol/l, at least 125 nmol/l, at least 150 nmol/l, at least 200 nmol/l, at least 250 nmol/l, at least 300 nmol/l or at least 350 nmol/l estradiol at a FSH concentration that is lower than the concentration necessary with human urinary FSH or recombinant FSH produced in CHO cells (Gonal F). Thus, it is preferably capable of releasing at least 50 nmol/l, at least 75 nmol/l, at least 100 nmol/l, at least 125 nmol/l, at least at least 150 nmol/l, at least 200 nmol/l, at least 250 nmol/l, 300 nmol/l or at least 350 nmol/l estradiol at a concentration wherein human urinary FSH or recombinant FSH produced in CHO cells (Gonal F) does not result in a corresponding, respectively equally high release of estradiol. As is demonstrated by the examples, the recombinant FSH preparations according to the present invention induce respectively stimulate the production of estradiol more strongly than human urinary FSH or recombinant FSH produced in CHO cells (Gonal F).

Accordingly, the recombinant FSH in the composition has one or more of the following characteristics as can be determined in a granulose cell assay

(a) it is capable of stimulating the release of progesterone in granulose ceils at concentrations which are below the minimum concentration needed for the induction of cAMP release by the granulose cells;
(b) it is capable of stimulating the release of at least 200ng/ml progesterone in about $5*10^4$ to about $1*10^5$ granulosa cells/ml at FSH concentrations which do not induce a cAMP release or which induce a cAMP release of less than 10 pmol/ml;
(c) it is capable of stimulating the release of at least 100ng/ml progesterone in about $5*10^4$ to about $1*10^5$ granulosa cells/ml at a concentration that is lower than the concentration needed by human urinary FSH or recombinant FSH produced in CHO cells (Gonal F); and/or
(d) it is capable of stimulating the release of at least 100ng/ml progesterone in about $5*10^4$ to about $1*10^5$ granulosa cells/ml at a concentration wherein human urinary FSH or recombinant FSH produced in CHO cells (Gonal F) do not result in a corresponding release of progesterone.

The respective characteristics described herein on the cAMP release and the expression of the sex steroids can be analysed and determined by using a granulose cell assay, as is e.g. described in the PCT patent application no. WO 2012/017058.

As discussed herein, in the recombinant FSH preparation according to the present invention may be capable of stimulating or co-stimulating the release of sex steroids such as progesterone, in particular the release of progesterone in granulosa cells, already at concentrations where no significant amount of cAMP is released, in particular, the recombinant FSH according to the present invention may be capable of stimulating release of sex steroids such as progesterone in granulosa cells at concentrations which are below the minimum concentration needed for the induction of cAMP release by the granulosa cells. Furthermore, in certain embodiments the recombinant FSH preparation according to the present invention is capable of stimulating release of progesterone, in particular release of progesterone in granulosa cells, by inducing a signal transduction pathway, which is independent of cAMP signaling. Preferably, the infertility treatment includes the induction of a signal transduction pathway, which is independent of cAMP signaling by the recombinant FSH according to the present invention, resulting in the stimulation of progesterone release. However, other signal transduction pathways including cAMP signaling may additionally be activated by the recombinant FSH. In other embodiments, the infertility treatment does not involve the induction of a significant release of cAMP by the recombinant FSH according to the present invention.

The term "infertility treatment" according to the invention means the treatment of a dysfunction or disease related to the reproduction or fertility of a human or animal subject, in particular, infertility treatment includes assisted reproductive technologies, ovulation induction, in-vitro fertilization, intrauterine insemination, as well as the enablement or improvement of germ cell maturation such as folliculogenesis and spermatogenesis.

In a preferred embodiment of the invention, the recombinant FSH in the composition is capable of inducing follicle growth in a female human being after administration of a single dose, wherein the single dose preferably comprises 25 to 500 IU FSH and preferably is administered parenteral, in particular by subcutaneous injection.

In a further preferred embodiment of the invention, the recombinant FSH is FSH-GEX.

The recombinant FSH composition according to the present invention preferably is present in a pharmaceutical composition. Thus, in a further embodiment of the present invention is a pharmaceutical composition comprising the recombinant FSH composition according to the present invention having a human glycosylation pattern. The pharmaceutical composition may be used in infertility treatment as defined herein. The pharmaceutical composition may include further pharmaceutically active agents, in particular further agents useful in infertility treatment such as other gonadotropins, in particular LH and/or CG, preferably recombinant and/or human LH or CG. Alternatively, the pharmaceutical composition comprising the recombinant FSH may be designed for use in combination with such further pharmaceutically active agents.

The term "pharmaceutical composition" particularly refers to a composition suitable for administering to a human or animal, i.e., a composition containing components, which are pharmaceutically acceptable. Preferably, a pharmaceutical composition comprises an active compound or a salt or prodrug thereof together with a carrier, diluent or pharmaceutical excipient such as buffer, preservative and tonicity modifier. The international units (IU) for FSH refer to the first international standard for urinary FSH WHO 92/512 (WHO Tech Rep. Series No.872, 1988) and are determined according to the augmented ovarian weight gain method (Steelman, S.L. & Pohley, F.M, (1953) Endocrinology 53, 604-616).

Furthermore, the present invention provides the recombinant FSH preparation according to the present invention or the pharmaceutical composition according to the present invention for use in infertility treatment as well as a method for treatment of infertility comprising the administration of the recombinant FSH preparation according to the present invention or the pharmaceutical composition according to the present invention to the patient.

The term "patient" means according to the invention a human being, a nonhuman primate or another animal, in particular a mammal such as a cow, horse, pig, sheep, goat, dog, cat or a rodent such as a mouse and rat. In a particularly preferred embodiment, the patient is a human being. In case of a human patient, the FSH preferably is human FSH. The patient may be male or female, and preferably is female.

It is also envisioned herein, the recombinant FSH preparation according to the invention is capable of eliciting a biological effect even after the administration of only a single dose. In particular, the FSH preparation according to the invention or the pharmaceutical composition according to the invention is capable of inducing follicular growth and/or ovular maturation in a patient, in particular a human patient, after administration of only a single dose of the FSH preparation or pharmaceutical composition. Preferably, the biological effect achieved after the administration of only a single dose is higher, in particular the follicular growth and/or ovular maturation, is more pronounced and/or is achieved in a higher ratio of the treated patients compared to FSH preparations obtained from human urine and/or expressed in CHO cells. Said single dose in particular comprises at least 10 IU FSH, preferably at least 15 IU FSH, at least 20 IU FSH or at least 25 IU FSH, and/or 1000 IU FSH or less, preferably 750 IU FSH or less, 500 IU FSH or less, 300 IU FSH or less, 200 IU FSH or less, 150 IU FSH or less, 100 IU FSH or less or 50 IU FSH or less. Preferably, said single dose comprises about 10 IU to about 500 IU FSH, more preferably about 20 IU to about 300 IU FSH, for example about 25 IU FSH, about 75 IU FSH or about 100 IU FSH.

It is envisioned herein that the recombinant FSH preparation according to the present invention is administered to the patient in a dose which results in a FSH concentration in the circulation of the patient of less than 10 IU/L. In certain embodiments, the dose to be administered to the patient results in an FSH concentration in the circulation of the patient which is less than about 8 IU/L, or less than about 6 IU/L, in particular less than about 5 IU/L, less than about 4 IU/L, less than about 3 IU/L or less than about 2 IU/L. The concentration of the FSH in the patient's circulation for example is in the range of about 0.2 to about 10 IU/L, in particular about 0.3 to about 8 IU/L, about 0.4 to about 7 IU/L or about 0.5 to about 1 IU/L. In particular, the FSH is administered to the patient in a dose, which does not induce a significant release of cAMP. However, the recombinant FSH preparations according to the invention may also be administered in a dose, which results in higher FSH concentrations in the patient's circulation. This may be the case if the FSH preparation is administered for several days. For example, the FSH preparation of the present invention may be administered between 2 and 20 days, preferably 4 and 18 days and even more preferably for 6 to 15 days. In case the FSH is administered for several days, a single dose may be about 150 IU or less, or about 125 IU or less, preferably about 100 IU or less, even more preferably about 75 IU or less. It is also envisioned that such a single dose may be about 50 IU or less or even 25 IU or less. The administration of the FSH of the present invention may for example result in a FSH concentration in the circulation of the patient of less than about 18 IU/L, or less than about 15 IU/L, or less than about 12 IU/L, in particular less than about 10 IU/L, less than about 8 IU/L or less than about 5 IU/L.

In further embodiments, as described herein, the recombinant FSH preparation according to the present invention is capable of stimulating or co-stimulating germ cell maturation by a biological process, which is independent of cAMP signaling.

Thus, the present invention also pertains to the recombinant FSH composition or the pharmaceutical composition described herein for inducing and/or stimulating the secretion of sex steroids also independent of cAMP. Furthermore, the present invention also pertains to the recombinant FSH composition or the pharmaceutical composition described above for stimulating or co-stimulating germ cell maturation by a biological process, which is independent of cAMP signaling. Additionally, the present invention also pertains to the recombinant FSH composition or the pharmaceutical composition described herein for inducing and/or stimulating the secretion of sex steroids at FSH concentrations at which no significant cAMP release is induced. Furthermore, the present invention also pertains to the recombinant FSH preparation or the pharmaceutical composition described herein for inducing sex steroid secretion at much lower concentrations than the commonly used urinary FSH or recombinant FSH obtained from CHO cells. The pharmacological and therapeutic advantages of the respective uses in particular for infertility treatment are discussed in detail herein.

"Sex steroids", also known as gonadal steroids or sex hormones, in particular refer to steroid hormones that interact with vertebrate androgen or estrogen receptors. The term "sex steroid" includes androgens such as anabolic steroids, androstenedione, dehydroepiandrosterone, dihydrotestosterone and testosterone; estrogens such as estradiol, estriol and estrone; and progesterone. Preferably, sex steroids refer to naturally occurring sex steroids, more preferably to natural human sex steroids. Preferred sex steroids according to the invention are estradiol and progesterone, in particular progesterone.

In particular, the infertility treatment may include the stimulation or co-stimulation of germ cell maturation by a biological process, which is independent of cAMP signaling. However, the infertility treatment may additionally comprise the stimulation of germ cell maturation by one or more other biological processes, which involve cAMP signaling, in other embodiments the infertility treatment does not involve the stimulation of germ cell maturation by such other biological processes.

The germ cell maturation preferably includes follicular growth and/or spermatogenesis. Furthermore, the biological process by which the FSH stimulates germ cell maturation may include secretion of sex steroids, in particular progesterone, preferably by granulosa cells. Preferably, the biological process which is independent of cAMP signaling refers to the secretion of sex steroids, in particular progesterone, preferably by granulosa cells, induced by a signal transduction pathway which does not involve cAMP as messenger molecule.

In preferred embodiments, infertility treatment includes assisted reproductive technologies, ovulation induction, in-vitro fertilization, for example in-vitro fertilization with intracytopiasmic sperm injection, gamete intrafallopian transfer, intrauterine insemination, treatment of anovulatory disorder in women, treatment of severe hormone deficiency disorder for egg maturation in woman, treatment of sperm production deficiencies in men, and/or the enablement or improvement of germ cell maturation such as foiliculogenesis and spermatogenesis, in particular follicle maturation in women, for example during in vitro fertilization stimulation protocols and/or for anovulatory disorder treatment.

In a further preferred embodiment of the invention the recombinant FSH composition or the pharmaceutical composition has one or more of the following characteristics:

(i) it is capable of inducing follicular growth and/or ovular maturation after the administration of only a single dose; and/or

(ii) it has a lower circulation half-life in one or more of humans, cynomolgus monkeys, rats and/or mice than FSH preparations obtained from human urine and/or expressed in CHO cells; and/or

(iii) it has a lower bioavailability in one or more of humans, cynomolgus monkeys, rats and/or mice than FSH preparations obtained from human urine and/or expressed in CHO cells; and/or

(iv) it has a therapeutic efficacy in one or more of humans, cynomolgus monkeys, rats and/or mice which is similar to or higher than that of FSH preparations obtained from human urine and/or expressed in CHO cells.

In further embodiments, the recombinant FSH preparation according to the invention has a lower circulation half-life than FSH preparations obtained from human urine and/or expressed in CHO cells. In particular, it has a lower circulation half-life in one or more of humans, cynomolgus monkeys, rats and/or mice. Preferably, the circulation half-life is at least 5% lower, more preferably at least 10%, at least 15% or at least 20% lower than that of FSH preparations obtained from human urine and/or expressed in CHO cells. In certain embodiments, the recombinant FSH preparation according to the invention has a lower bioavailability than FSH preparations obtained from human urine and/or expressed in CHO cells, in particular, in one or more of humans, cynomolgus monkeys, rats and/or mice. Preferably, the bioavailability is at least 5% lower, more preferably at least 10%, at least 15% or at least 20% lower than that of FSH preparations obtained from human urine and/or expressed in CHO cells. Bioavailability in this respect preferably refers to the area under the curve (AUG) value obtained in pharmacokinetic studies wherein the serum FSH concentration is determined at different time points after administration of a defined amount of FSH. Circulation half-life and bioavailability preferably are determined after administration of the FSH by subcutaneous injection, in particular after single dose administration, wherein the single dose preferably comprises about 10 to about 1000 IU FSH, more preferably about 25 IU to about 500 IU FSH or about 50 IU to about 300 IU FSH, in particular about 100 IU FSH. In particular, circulation half-life and bioavailability are determined as disclosed in the PCT patent application no. WO 2012/017058.

In preferred embodiments, the recombinant FSH composition or the pharmaceutical composition according to the invention has a therapeutic efficacy which is similar to or even higher than that of FSH compositions or pharmaceutical compositions obtained from human urine and/or expressed in CHO cells, in particular, in one or more of humans, cynomolgus monkeys, rats and/or mice. The term "therapeutic efficacy" preferably refers to the ability to stimulate release of estradiol and/or inhibin-B when administered to a subject. The therapeutic efficacy preferably is determined by measuring the estradiol and/or inhibin-B concentration in the blood or serum of one or more subjects after administration of the FSH by subcutaneous injection, in particular after single dose administration, wherein the single dose preferably comprises about 10 to about 1000 IU FSH, preferably about 25 IU to about 500 IU FSH or about 50 IU to about 300 IU FSH, in particular about 100 IU FSH. In particular, the therapeutic efficacy is determined as disclosed in the PCT patent application no. WO 2012/017058. Similar therapeutic efficacies in particular refer to stimulations of estradiol and/or inhibin-B release by the respective FSH preparations which result in estradiol and/or inhibin-B serum concentrations which differ from each other by no more than 25%, preferably no more than 20%, no more than 15% or no more than 10%.

The pharmaceutical composition according to the invention may be in the form of a single unit dose or a multiple unit dose. Preferably, the pharmaceutical composition is a sterile solution comprising the recombinant FSH according to the present invention, further comprising one or more ingredients selected from the group consisting of solvents such as water, buffer substances, stabilizers, preservatives, excipients, surfactants and salts. A single unit dose preferably comprises about 10 IU to about 750 IU FSH, more preferably about 25 IU to about 500 IU FSH, about 50 IU to about 400 IU FSH, or about 100 IU to about 300 IU FSH. A multiple unit dose comprises enough FSH to provide for multiple single doses, in particular at least 5, at least 10, at least 20 or at least 50 single doses. The pharmaceutical composition may for example be in the form of an injection pen.

Preferably, the recombinant FSH preparation according to the present invention is for parenteral administration to the patient. In particular, the recombinant FSH is to be administered by injection or infusion, for example intravenously, intramuscularly or subcutaneously. In certain embodiments of the present invention, the recombinant FSH is present in a pharmaceutical composition. Suitable dosage regiments can be determined by the skilled artisan and can be derived from the general knowledge in the field.

The FSH composition obtained from human urine in particular is obtained from urine of post-menopause women. The FSH preparation expressed in CHO cells is for example expressed in the CHO cell line CHOdhfr- [ATCC No. CRL-9096]. The FSH preparation obtained from human urine and the FSH preparation expressed in CHO cells preferably are commercially available and approved pharmaceutical preparations, in particular Bravelle and Gonal-f, respectively. When comparing the effects of different FSH preparations, in particular their circulation half-life, bioavailability and therapeutic efficacy, the FSH preparations are analyzed by administering them to similar subject groups with the same dosage regimen using the same administration pathway and using similar or the same further conditions.

Furthermore, it is envisioned that the FSH composition or the pharmaceutical composition according of the present invention may be used for the preparation of a pharmaceutical composition for the induction of follicle growth and/or ovular maturation, and/or a use as defined herein.

### Examples

The following Examples illustrate the invention, but are not to be construed as limiting the scope of the invention.

### Example 1: Activity determination of FSH in presence and absence of Chlorocresol

FSH-GEX™ binds to the hFSHR expressed on stably transfected HEK293 cells and elicits the production of cAMP. For stimulation assays HEK293-FSH-R cells were harvested and seeded into 24 well plates at a density of $5\times10^4$ cells/ml (500$\mu$l/well). Cells were cultured overnight in DMEM containing 10% FCS, 2% L-glutamine and 1 $\mu$M androstendione at 37°C and 8% $CO_2$. Subsequently, supernatant was removed, adherent cells were washed with DMEM and FSH-GEX™ with concentrations ranging from 1500 to 1.46 ng/ml was added. Serial dilution of FSH-GEX™ was done in DMEM containing 10% FCS, 2% L-glutamine and 1 $\mu$M androstendione. All samples were run in duplicates. After 3h incubation at 37°C and 8% $CO_2$ 300 $\mu$l of supernatant were collected and stored at -80°C until further analyzing. The remaining cells were incubated for another 21 h at 37°C and 8% $CO_2$. After a total of 24 h the plates were frozen at -80°C. Cell lysates were prepared by thawing and pipetting up and down in the remaining supernatant. Lysates from duplicate samples were pooled and cleared by centrifugation (1200 rpm (311xg), 5 min).

To determine the cAMP content the supernatant was analyzed by a commercial competitive EIA (cyclic AMP - Enzyme Immunoassay Kit, Enzo life sciences). The assay was performed according to the manufacturer's protocol. Samples were used non-acetylated without further dilution. The duplicate wells from the stimulation assay were measured as singlets in the EIA. Absorbance at 450 nm was measured with a microplate reader (infinite F200, TECAN) and noise/signal ratio was calculated to evaluate the cAMP release as a marker for stimulation of the cells. Noise/signal ratios larger than one indicate a cAMP release of the cells. The higher the noise/signal ratio the more cAMP was released. Based on the dose response curve an $EC_{50}$ is determined and the potency is obtained by dividing the $EC_{50}$ from the test sample (in this case chlorocresol containing sample) to the reference material (in this case sample without chlorocresol). The test sample is considered to be fully active when a potency of >0.8 to <1.2 is observed at the respective test point.

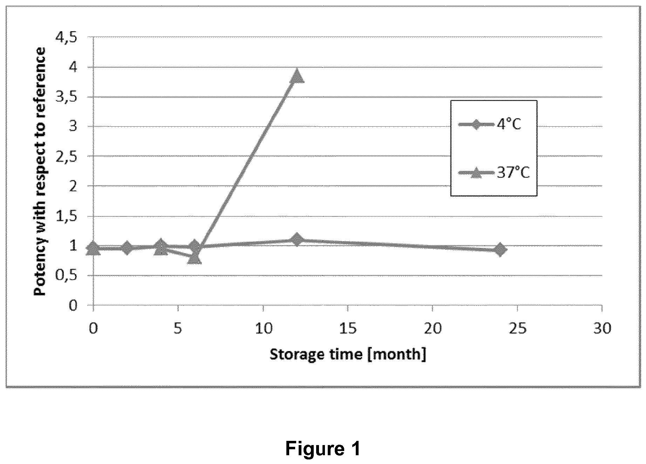

The results of the activity determination over 24 month at 4°C illustrate the full biological activity of FSH in presence of chlorocresol over the full testing period (see Figure 1). Furthermore, when the storage temperature is increased to 37°C the activity of the control sample reduced compared to the chlorocresol containing sample indicating a stabilization effect of the latter (see Figure 2).

### Example 2: Dynamic Light Scattering (DLS), melting point

The melting point was determined by dynamic light scattering using a Zetasizer Nano ZS ZEN3600 (Malvern Instruments). Twenty $\mu$L of sample was filled in a 12 $\mu$L cuvette (Low volume quartz cuvette ZEN2112, cat# 105.251.006-QS, Hellma). Measurement parameters in the DTS v5.1 Control Software were: "Mark-Houwink parameters", material "protein" (RI 1.45), buffer "50 mg/mL sucrose" (calculated viscosity 1.1685-0.4754 cP, depending on temperature), "multiple narrow modes", "seek for optimum position", "automatic attenuation selection", run time "automatic", number of runs 1, number of measurements 1. The temperature was increased from 20 to 90 °C in 2 degrees increments. Prior to measurement the cuvette was equilibrated for 2 min at each temperature step. The detection angle of the scattered light was 173° (backward scatter). The measured Z-average (diameter in nm) was evaluated in dependence on the increasing temperature.

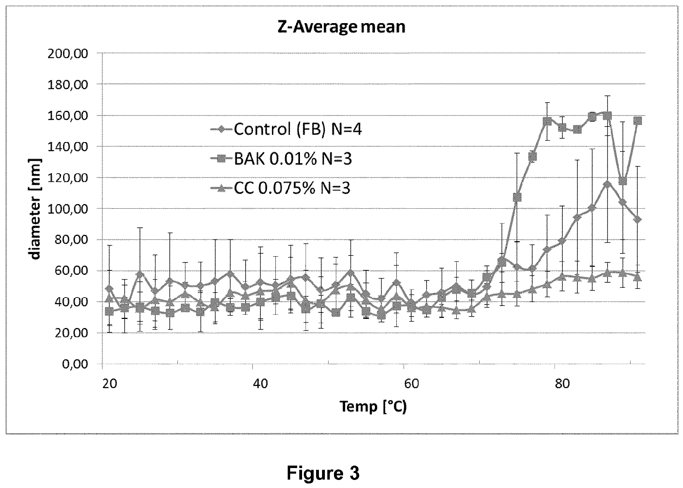

The results are given in Figure 3. In contrast to the formulation without preservative or benzalkoniumchloride the preservative chlorocresol stabilizes the FSH molecule as can be seen by the lowest increase in Z-average in dependence of the temperat

**Claims**

1. A composition comprising recombinant FSH and chlorocresol, wherein the recombinant FSH has a human glycosylation pattern.

2. The composition according to claim 1, wherein the recombinant FSH has an increased stability at high temperatures compared to a recombinant FSH with a mammalian glycosylation pattern comprised in a composition without chlorocresol, wherein the high temperature is preferably 37°C or more.

3. The composition according to any one of the preceding claims further comprising a surfactant a tonicity modifier, a buffering agent, a stabilizer and/or an excipient, wherein the surfactant is preferably Poloxamer 188 and wherein the stabilizer is preferably L-methionine.

4. The composition according to any one of the preceding claims, wherein the recombinant FSH in the composition has a glycosylation pattern comprising one or more of the following characteristics:

   (i) a relative amount of glycans carrying bisecting N-acetylglucosamine (bisGlcNAc) of at least 20%;
   (ii) a relative amount of glycans carrying fucose of at least 30%;
   (iii) a relative amount of 2,6-coupled sialic acid of at least 30%; or
   (iv) it is a diverse glycosylation pattern,

   wherein the glycosylation pattern preferably comprises at least two of the features (i), (ii) and (iii), and more preferably all of the features (i), (ii) and (iii).

5. The composition according to any one of the preceding claims, wherein the recombinant FSH is obtainable by production

   (i) in the human cell line GT-5s or a cell line derived therefrom or a cell line homologous thereto; or
   (ii) in the human cell line PerC6.

6. The composition according to any one of the preceding claims, wherein the recombinant FSH in the composition comprises one or more of the following characteristics:

   (a) the glycosylation pattern comprises a relative amount of glycans carrying one or more sialic acid residues of at least 85%;
   (b) the glycosylation pattern comprises a relative amount of at least tetraantennary glycans of at least 18%;
   (c) a Z-number of at least 200;
   (d) it is human recombinant FSH; or
   (e) it is produced by a human cell line or human cells.

7. The composition according to any one of the preceding claims, wherein the recombinant FSH in the composition has a glycosylation pattern comprising one or more of the following characteristics:

   (i) a relative amount of glycans carrying bisecting N-acetylglucosamine (bisGlcNAc) in the range of from 25% to 50%;
   (ii) a relative amount of at least tetraantennary glycans of at least 16%;
   (iii) a relative amount of glycans carrying fucose of at least 35%;
   (iv) a relative amount of 2,6-coupled sialic acid of at least 53%;

(v) a relative amount of glycans carrying one or more sialic acid residues of at least 88%;
(vi) a Z-number of at least 220;
(vii) a relative amount of glycans carrying galactose of at least 95%;
(viii) a relative amount of glycan branches carrying a terminal galactose unit optionally modified by a sialic acid residue of at least 60%;
(ix) a relative amount of glycans carrying a sulfate group of at least 3%;
(x) it comprises at least 45 different glycan structures, wherein each one of the different glycan structures has a relative amount of at least 0.05% of the total amount of glycan structures of the FSH in the composition;
(xi) it comprises at least 35 different glycan structures, wherein each one of the different glycan structures has a relative amount of at least 0.1% of the total amount of glycan structures of the FSH in the composition;
(xii) it comprises at least 20 different glycan structures, wherein each one of the different glycan structures has a relative amount of at least 0.5% of the total amount of glycan structures of the FSH in the composition; or
(xiii) it comprises at least 40% more different glycan structures than FSH obtained from CHO cells in a corresponding composition, wherein each one of the different glycan structures has a relative amount of at least 0.05% of the total amount of glycan structures of the FSH in the respective composition.

8.  The composition according to any one of the preceding claims, wherein the recombinant FSH in the composition has a glycosylation pattern comprising the following characteristics:

(i) a relative amount of glycans carrying bisecting N-acetylglucosamine (bisGlcNAc) in the range of from 25% to 50%;
(ii) a relative amount of at least tetraantennary glycans of at least 16%;
(iii) a relative amount of glycans carrying fucose of at least 35%;
(iv) a relative amount of 2,6-coupled sialic acid in the range of from 53% to 99%; and
(v) a relative amount of glycans carrying one or more sialic acid residues of at least 88%.

9.  The composition according to any one of the preceding claims, wherein the recombinant FSH in the composition is capable of stimulating the release of progesterone in granulosa cells

(a) at concentrations where no significant amounts of cAMP are released; and/or
(b) by inducing a signal transduction pathway which is independent of cAMP signaling; and/or

wherein the recombinant FSH in the composition is capable of stimulating or co-stimulating germ cell maturation by a biological process which is independent of cAMP signaling.

10. The composition according to any one of the preceding claims, wherein the recombinant FSH in the composition has one or more of the following characteristics as can be determined in a granulose cell assay

(a) it is capable of stimulating the release of progesterone in granulose cells at concentrations which are below the minimum concentration needed for the induction of cAMP release by the granulose cells;
(b) it is capable of stimulating the release of at least 200 ng/ml progesterone in $5*10^4$ to $1*10^5$ granulosa cells/ml at FSH concentrations which do not induce a cAMP release or which induce a cAMP release of less than 10 pmol/ml;
(c) it is capable of stimulating the release of at least 100 ng/ml progesterone in $5*10^4$ to $1*10^5$ granulosa cells/ml at a concentration that is lower than the concentration needed by human urinary FSH or recombinant FSH produced in CHO cells (Gonal F); and/or
(d) it is capable of stimulating the release of at least 100 ng/ml progesterone in $5*10^4$ to $1*10^5$ granulosa cells/ml at a concentration wherein human urinary FSH or recombinant FSH produced in CHO cells (Gonal F) do not result in a corresponding release of progesterone.

11. The composition according to any one of the preceding claims, wherein the recombinant FSH in the composition is capable of inducing follicle growth in a female human being after administration of a single dose, wherein the single dose preferably comprises 25 to 500 IU FSH and preferably is administered parenteral, in particular by subcutaneous injection.

12. The composition according to any one of the preceding claims, wherein the recombinant FSH is FSH-GEX or wherein the composition is a pharmaceutical composition.

13. The composition according to any one of claims 1 to 12 or the pharmaceutical composition according to claim 12 for use in infertility treatment.

14. The composition or pharmaceutical composition for use according to claim 13, wherein

(i) the dose to be administered to the patient results in an FSH concentration in the circulation of the patient in the range of 0.2 to 10 IU/L, preferably 0.4 to 7 IU/L
(ii) infertility treatment comprises inducing and/or stimulating the secretion of sex steroids independent of cAMP;
(iii) infertility treatment comprises stimulating or co-stimulating germ cell maturation by a biological process independent of cAMP signaling;
(iv) infertility treatment comprises inducing and/or stimulating the secretion of sex steroids at FSH concentrations at which no significant cAMP release is induced; and/or
(v) wherein the infertility treatment includes assisted reproductive technologies, ovulation induction, in-vitro fertilization, for example in-vitro fertilization with intracytoplasmic sperm injection, gamete intrafallopian transfer, intrauterine insemination, treatment of anovulatory disorder in women, treatment of severe hormone deficiency disorder for egg maturation in woman, treatment of sperm production deficiencies in men, and/or the enablement or improvement of germ cell maturation such as folliculogenesis and spermatogenesis, in particular follicle maturation in women, for example during in vitro fertilization stimulation protocols and/or for anovulatory disorder treatment.

15. The composition or the pharmaceutical composition for use according to claim 13, wherein the infertility treatment involves the induction of follicle growth and/or ovular maturation.


**Patentansprüche**

1. Zusammensetzung umfassend rekombinantes FSH und Chlorocresol, wobei das rekombinante FSH ein humanes Glykosylierungsmuster aufweist.

2. Zusammensetzung nach Anspruch 1, wobei das rekombinante FSH eine erhöhte Stabilität bei hohen Temperaturen im Vergleich zu einem rekombinanten FSH mit einem Säugetierglykosylierungsmuster aufweist, umfasst in einer Zusammensetzung ohne Chlorocresol, wobei die hohe Temperatur vorzugsweise 37°C oder mehr beträgt.

3. Zusammensetzung nach einem der vorhergehenden Ansprüche, ferner umfassend ein Tensid einen Tonizitätsmodifikator, ein Puffermittel, einen Stabilisator und/oder einen Hilfsstoff, wobei das Tensid vorzugsweise Poloxamer 188 und wobei der Stabilisator vorzugsweise L-Methionin ist.

4. Zusammensetzung nach einem der vorhergehenden Ansprüche, wobei das rekombinante FSH in der Zusammensetzung ein Glykosylierungsmuster aufweist umfassend eines oder mehrere der folgenden Merkmale:

(i) eine relative Menge an Glykanen, die bisektierendes N-Acetylglucosamin (bisGlcNAc) von mindestens 20% tragen;
(ii) eine relative Menge an Glykanen, die Fucose von mindestens 30% tragen;
(iii) eine relative Menge an 2,6-gekoppelter Sialinsäure von mindestens 30%; oder
(iv) es handelt sich um ein vielfältiges Glykosylierungsmuster,

wobei das Glykosylierungsmuster vorzugsweise mindestens zwei der Merkmale (i), (ii) und (iii) und noch bevorzugter alle Merkmale (i), (ii) und (iii) umfasst.

5. Zusammensetzung nach einem der vorhergehenden Ansprüche, wobei das rekombinante FSH erhältlich ist durch Herstellung

(i) in der menschlichen Zelllinie GT-5s oder einer davon abgeleiteten Zelllinie oder einer homologen Zelllinie davon; oder
(ii) in der menschlichen Zelllinie PerC6.

6. Zusammensetzung nach einem der vorhergehenden Ansprüche, wobei das rekombinante FSH in der Zusammensetzung eines oder mehrere der folgenden Merkmale umfasst:

(a) das Glykosylierungsmuster umfasst eine relative Menge an Glykanen, die einen oder mehrere Sialinsäurereste von mindestens 85% tragen;

(b) das Glykosylierungsmuster umfasst eine relative Menge von mindestens tetraantennären Glykanen von mindestens 18%;

(c) eine Z-Nummer von mindestens 200;

(d) es handelt sich um menschliches rekombinantes FSH; oder

(e) es wird produziert von einer menschlichen Zelllinie oder von menschlichen Zellen.

7. Zusammensetzung nach einem der vorhergehenden Ansprüche, wobei das rekombinante FSH in der Zusammensetzung ein Glykosylierungsmuster aufweist umfassend eines oder mehrere der folgenden Merkmale:

(i) eine relative Menge an Glykanen, die bisektierendes N-Acetylglucosamin (bisGlcNAc) im Bereich von 25% bis 50% tragen;

(ii) eine relative Menge von mindestens tetraantennären Glykanen von mindestens 16%;

(iii) eine relative Menge an Glykanen, die Fucose von mindestens 35% tragen;

(iv) eine relative Menge an 2,6-gekoppelter Sialinsäure von mindestens 53%;

(v) eine relative Menge an Glykanen, die einen oder mehrere Sialinsäurerückstände von mindestens 88% tragen;

(vi) eine Z-Nummer von mindestens 220;

vii) eine relative Menge an Glykanen, die Galaktose tragen, von mindestens 95%;

(viii) eine relative Menge an Glykanzweigen, die eine terminale Galaktoseeinheit tragen, gegebenenfalls modifiziert durch einen Sialinsäurerückstand von mindestens 60%;

(ix) eine relative Menge an Glykanen, die eine Sulfatgruppe von mindestens 3% tragen;

(x) es umfasst mindestens 45 verschiedene Glykanstrukturen, wobei jede der verschiedenen Glykanstrukturen eine relative Menge von mindestens 0,05% der Gesamtmenge an Glykanstrukturen des FSH in der Zusammensetzung aufweist;

(xi) es umfasst mindestens 35 verschiedene Glykanstrukturen, wobei jede der verschiedenen Glykanstrukturen eine relative Menge von mindestens 0,1% der Gesamtmenge an Glykanstrukturen des FSH in der Zusammensetzung aufweist;

(xii) es umfasst mindestens 20 verschiedene Glykanstrukturen, wobei jede der verschiedenen Glykanstrukturen eine relative Menge von mindestens 0,5% der Gesamtmenge an Glykanstrukturen des FSH in der Zusammensetzung aufweist; oder

(xiii) es umfasst mindestens 40% mehr verschiedene Glykanstrukturen als FSH erhalten aus CHO-Zellen in einer entsprechenden Zusammensetzung, wobei jede der verschiedenen Glykanstrukturen eine relative Menge von mindestens 0,05% der Gesamtmenge an Glykanstrukturen des FSH in der jeweiligen Zusammensetzung aufweist.

8. Zusammensetzung nach einem der vorhergehenden Ansprüche, wobei das rekombinante FSH in der Zusammensetzung ein Glykosylierungsmuster aufweist umfassend die folgenden Merkmale:

(i) eine relative Menge an Glykanen, die bisektierendes N-Acetylglucosamin (bisGlcNAc) im Bereich von 25% bis 50% tragen;

(ii) eine relative Menge von mindestens tetraantennären Glykanen von mindestens 16%;

(iii) eine relative Menge an Glykanen, die Fucose von mindestens 35% tragen;

(iv) eine relative Menge an 2,6-gekoppelter Sialinsäure im Bereich von 53% bis 99%; und

(v) eine relative Menge an Glykanen, die einen oder mehrere Sialinsäurerückstände von mindestens 88% tragen.

9. Zusammensetzung nach einem der vorhergehenden Ansprüche, wobei das rekombinante FSH in der Zusammensetzung in der Lage ist, die Freisetzung von Progesteron in Granulosazellen zu stimulieren

(a) in Konzentrationen, in denen keine signifikanten Mengen an cAMP freigesetzt werden; und/oder

(b) durch Induktion eines Signaltransduktionswegs, der von der cAMP-Signalübertragung unabhängig ist; und/oder

wobei das rekombinante FSH in der Zusammensetzung in der Lage ist, die Keimzellreifung durch einen biologischen Prozess, der unabhängig von der cAMP-Signalübertragung ist, zu stimulieren oder co-zu stimulieren.

10. Zusammensetzung nach einem der vorhergehenden Ansprüche, wobei das rekombinante FSH in der Zusammensetzung eines oder mehrere der folgenden Merkmale aufweist, wie in einem Granulosezellassay festgestellt werden kann

(a) es ist in der Lage, die Freisetzung von Progesteron in Granulosezellen in Konzentrationen zu stimulieren, die unter der Mindestkonzentration liegen, die für die Induktion der cAMP-Freisetzung durch die Granulosezellen benötigt werden;

(b) es ist in der Lage, die Freisetzung von mindestens 200 ng/ml Progesteron in $5*10^4$ bis $1*10^5$ Granulosazellen/ml bei FSH-Konzentrationen zu stimulieren, die keine cAMP-Freisetzung induzieren oder die eine cAMP-Freisetzung von weniger als 10 pmol/ml induzieren;

(c) es ist in der Lage, die Freisetzung von mindestens 100 ng/ml Progesteron in $5*10^4$ bis $1*10^5$ Granulosazellen/ml in einer Konzentration zu stimulieren, die niedriger ist als die Konzentration, die von menschlichem FSH im Urin oder rekombinantem FSH produziert in CHO-Zellen (Gonal F) benötigt wird, und/oder

(d) es ist in der Lage, die Freisetzung von mindestens 100 ng/ml Progesteron in $5*10^4$ bis $1*10^5$ Granulosazellen/ml in einer Konzentration zu stimulieren, wobei menschliches FSH im Urin oder rekombinantes FSH produziert in CHO-Zellen (Gonal F) nicht zu einer entsprechenden Freisetzung von Progesteron führt.

11. Zusammensetzung nach einem der vorhergehenden Ansprüche, wobei das rekombinante FSH in der Zusammensetzung in der Lage ist, in einem weiblichen Menschen nach Verabreichung einer Einzeldosis, Follikelwachstum zu induzieren, wobei die Einzeldosis vorzugsweise 25 bis 500 IE FSH umfasst und vorzugsweise parenteral, insbesondere durch subkutane Injektion, verabreicht wird.

12. Zusammensetzung nach einem der vorhergehenden Ansprüche, wobei das rekombinante FSH FSH-GEX ist oder wobei die Zusammensetzung eine pharmazeutische Zusammensetzung ist.

13. Zusammensetzung nach einem der Ansprüche 1 bis 12 oder pharmazeutische Zusammensetzung nach Anspruch 12 zur Verwendung bei der Behandlung von Unfruchtbarkeit.

14. Zusammensetzung oder pharmazeutische Zusammensetzung zur Verwendung nach Anspruch 13, wobei

(i) die dem Patienten zu verabreichende Dosis zu einer FSH-Konzentration im Kreislauf des Patienten im Bereich von 0,2 bis 10 IE/L führt, vorzugsweise 0,4 bis 7 IE/L

(ii) die Behandlung von Unfruchtbarkeit Induzieren und/oder Stimulieren der Sekretion von Sexualsteroiden unabhängig von cAMP umfasst;

(iii) die Behandlung von Unfruchtbarkeit Stimulieren oder Co-Stimulieren der Keimzellreifung durch einen biologischen Prozess unabhängig von der cAMP-Signalübertragung umfasst;

(iv) die Behandlung von Unfruchtbarkeit Induzieren und/oder Stimulieren der Sekretion von Sexualsteroiden in FSH-Konzentrationen umfasst, bei denen keine signifikante cAMP-Freisetzung induziert wird; und/oder

(v) wobei die Behandlung von Unfruchtbarkeit assistierte Reproduktionstechnologien, Ovulationsinduktion, in-vitro-Fertilisation, zum Beispiel in-vitro-Fertilisation mit intrazytoplasmatischer Spermieninjektion, Gameten-Intrafallopian-Transfer, intrauterine Insemination, Behandlung von anovulatorischer Erkrankung bei Frauen, Behandlung von schwerer Hormonmangelerkrankung für Eizellreifung bei Frauen, Behandlung von Spermienproduktionsmängeln bei Männern und/oder die Ermöglichung oder Verbesserung der Keimzellreifung, wie Follikulogenese und Spermatogenese, insbesondere Follikelreifung bei Frauen, beispielsweise während in-vitro-Fertilisation Stimulationsprotokollen und/oder zur Behandlung von anovulatorischer Erkrankungumfasst.

15. Zusammensetzung oder pharmazeutische Zusammensetzung zur Verwendung nach Anspruch 13, wobei die Behandlung von Unfruchtbarkeit die Induktion des Follikelwachstums und/oder der ovulären Reifung beinhaltet.

**Revendications**

1. Composition comprenant de la FSH recombinante et du chlorocrésol, dans laquelle la FSH recombinante présente un schèma de glycosylation humain.

2. Composition selon la revendication 1, dans laquelle la FSH recombinante présente une stabilité accrue à haute température par rapport à une FSH recombinante avec un schèma de glycosylation mammalien comprise dans une composition sans chlorocrésol, la température élevée étant de préférence de 37°C ou plus.

3. Composition selon l'une des revendications précédentes, comprenant en outre un tensioactif, un modificateur de tonicité, un agent tampon, un stabilisateur et/ou un excipient, le tensioactif est de préférence le Poloxamer 188 et le stabilisateur étant de préférence la L-méthionine.

4. Composition selon l'une des revendications précédentes, dans laquelle la FSH recombinante dans la composition a un schéma de glycosylation comprenant une ou plusieurs des caractéristiques suivantes :

(i) une quantité relative de glycanes portant de la N-acétylglucosamine bissectrice (bisGlcNAc) d'au moins 20%;
(ii) une quantité relative de glycanes portant du fucose d'au moins 30%;
(iii) une quantité relative d'acide sialique couplé 2,6 d'au moins 30% ; ou
(iv) il s'agit d'un schéma de glycosylation diversifié,

dans lequel le motif de glycosylation comprend de préférence au moins deux des caractéristiques (i), (ii) et (iii), et de préférence toutes les caractéristiques (i), (ii) et (iii).

5. Composition selon l'une des revendications précédentes, dans laquelle la FSH recombinante peut être obtenue par production

(i) dans la lignée cellulaire humaine GT-5s ou une lignée cellulaire dérivée de celle-ci ou une lignée cellulaire homologue ; ou
(ii) dans la lignée cellulaire humaine PerC6.

6. Composition selon l'une des revendications précédentes, dans laquelle la FSH recombinante dans la composition comprend une ou plusieurs des caractéristiques suivantes:

(a) le schéma de glycosylation comprend une quantité relative de glycanes portant un ou plusieurs résidus d'acide sialique d'au moins 85%;
(b) le schéma de glycosylation comprend une quantité relative d'au moins 18 % de glycanes tétraantennaires;
(c) un nombre Z d'au moins 200;
(d) il s'agit d'une FSH recombinante humaine; ou
(e) elle est produite par une lignée cellulaire humaine ou des cellules humaines.

7. Composition selon l'une des revendications précédentes, dans laquelle la FSH recombinante dans la composition un schéma de glycosylation comprenant une ou plusieurs des caractéristiques suivantes :

(i) une quantité relative de glycanes portant la N-acétylglucosamine bissectrice (bisGlcNAc) comprise entre 25% et 50%;
(ii) une quantité relative de glycanes au moins tétraantennaires d'au moins 16%;
(iii) une quantité relative de glycanes portant de fucose d'au moins 35%;
(iv) une quantité relative d'acide sialique couplé à 2,6% d'au moins 53%;
(v) une quantité relative de glycanes portant un ou plusieurs résidus d'acide sialique d'au moins 88%;
(vi) un nombre Z d'au moins 220;
(vii) une quantité relative de glycanes portant de galactose d'au moins 95%;
(viii) une quantité relative de branches de glycanes portant une unité terminale de galactose éventuellement modifiée par un résidu d'acide sialique d'au moins 60%;
(ix) une quantité relative de glycanes portant un groupe sulfate d'au moins 3%;
(x) il comprend au moins 45 structures de glycanes différentes, chacune des structures de glycanes différentes ayant une quantité relative d'au moins 0,05% de la quantité totale de structures glycanes de la FSH dans la composition;
(xi) il comprend au moins 35 structures de glycanes différentes, chacune des structures glycanes différentes ayant une quantité relative d'au moins 0,1% de la quantité totale de structures glycanes de la FSH dans la composition;
(xii) il comprend au moins 20 structures glycanes différentes, dans lequelles chacune des structures glycanes différentes ayant une quantité relative d'au moins 0,5% de la quantité totale de structures glycanes de la FSH dans la composition; ou
(xiii) elle comprend au moins 40% de structures glycanes différentes de plus que la FSH obtenue à partir de cellules CHO dans une composition correspondante, chacune des structures glycanes différentes ayant une quantité relative d'au moins 0,05% de la quantité totale de structures glycanes de la FSH dans la composition respective.

8. Composition selon l'une des revendications précédentes, dans laquelle la FSH recombinante dans la composition a un schéma de glycosylation comprenant les caractéristiques suivantes :

(i) une quantité relative de glycanes portant la N-acétylglucosamine bissectrice (bisGlcNAc) comprise entre 25% et 50%;
(ii) une quantité relative de glycanes au moins tétraantennaires d'au moins 16%;
(iii) une quantité relative de glycanes portant du fucose d'au moins 35%;
(iv) une quantité relative d'acide sialique couplé au 2,6 comprise entre 53% et 99%; et
(v) une quantité relative de glycanes portant un ou plusieurs résidus d'acide sialique d'au moins 88%.

9. Composition selon l'une des revendications précédentes, dans laquelle la FSH recombinante dans la composition est capable de stimuler la libération de progestérone dans les cellules de la granulosa

(a) à des concentrations où aucune quantité importante d'AMPc n'est libérée; et/ou
(b) en induisant une voie de transduction du signal qui est indépendante de la signalisation de l'AMPc; et/ou

dans laquelle la FSH recombinante contenue dans la composition est capable de stimuler ou de co-stimuler la maturation des cellules germinales par un processus biologique indépendant de la signalisation de l'AMPc.

10. Composition selon l'une des revendications précédentes, dans laquelle la FSH recombinante dans la composition présente une ou plusieurs des caractéristiques suivantes qui peuvent être déterminées dans un essai sur cellules de granulose

(a) il est capable de stimuler la libération de progestérone dans les cellules de granulose à des concentrations inférieures à la concentration minimale nécessaire pour l'induction de la libération d'AMPc par les cellules de granulose;
(b) il est capable de stimuler la libération d'au moins 200 ng/ml de progestérone dans $5*10^4$ à $1*10^5$ cellules de granulosa/ml à des concentrations de FSH qui n'induisent pas de libération d'AMPc ou qui induisent une libération d'AMPc inférieure à 10 pmol/ml;
(c) il est capable de stimuler la libération d'au moins 100 ng/ml de progestérone dans $5*10^4$ à $1*10^5$ cellules de la granulosa/ml à une concentration inférieure à la concentration nécessaire pour la FSH urinaire humaine ou la FSH recombinante produite dans les cellules CHO (Gonal F); et/ou
(d) il est capable de stimuler la libération d'au moins 100 ng/ml de progestérone dans $5*10^4$ à $1*10^5$ cellules de la granulosa/ml à une concentration où la FSH urinaire humaine ou la FSH recombinante produite dans les cellules CHO (Gonal F) n'entraîne pas une libération correspondante de progestérone.

11. Composition selon l'une des revendications précédentes, dans laquelle la FSH recombinante contenue dans la composition est capable d'induire une croissance folliculaire chez une femme après l'administration d'une dose unique, la dose unique comprenant de préférence 25 à 500 UI de FSH et étant administrée de préférence par voie parentérale, en particulier par injection sous-cutanée.

12. Composition selon l'une des revendications précédentes, dans laquelle la FSH recombinante est la FSH-GEX ou dans laquelle la composition est une composition pharmaceutique.

13. Composition selon l'une des revendications 1 à 12 ou composition pharmaceutique selon la revendication 12 pour utilisation dans le traitement de l'infertilité.

14. Composition ou composition pharmaceutique à utiliser selon la revendication 13, dans laquelle

(i) la dose à administrer au patient entraîne une concentration de FSH dans la circulation du patient comprise entre 0,2 et 10 UI/L, de préférence entre 0,4 et 7 UI/L
(ii) le traitement de la stérilité comprend l'induction et/ou la stimulationde la sécrétion de stéroïdes sexuels indépendants de l'AMPc ;
(iii) le traitement de l'infertilité comprend la stimulation ou la co-stimulation de la maturation des cellules germinales par un processus biologique indépendant de la signalisation de l'AMPc ;
(iv) le traitement de l'infertilité comprend l'induction et/ou la stimulation de la secretion de stéroïdes sexuels à des concentrations de FSH qui n'induisent pas de libération significative d'AMPc ; et/ou
(v) dans traitement de l'infertilité comprend les techniques de procréation assistée, l'induction de l'ovulation, la fécondation in vitro, par exemple la fécondation in vitro avec injection intracytoplasmique de spermatozoïdes, le transfert intrafallope de gamètes, l'insémination intra-utérine, le traitement du trouble anovulatoire chez la femme, le traitement du trouble grave de la déficience hormonale pour la maturation des ovules chez la femme, le traitement des déficiences de la production de spermatozoïdes chez l'homme, et/ou la facilitation ou l'amélioration de la maturation des cellules germinales telles que la folliculogenèse et la spermatogenèse, en particulier la maturation folliculaire chez la femme, par exemple pendant les protocoles de stimulation de la fécondation in vitro et/ou pour le traitement des troubles anovulatoires.

15. Composition ou composition pharmaceutique à utiliser selon la revendication 13, dans laquelle le traitement de l'infertilité implique l'induction de la croissance folliculaire et/ou de la maturation ovulaire.

**Figure 1**

**Figure 2**

**Figure 3**

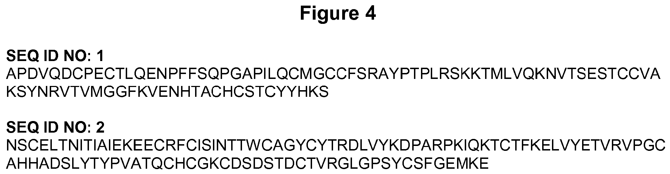

# Figure 4

**SEQ ID NO: 1**
APDVQDCPECTLQENPFFSQPGAPILQCMGCCFSRAYPTPLRSKKTMLVQKNVTSESTCCVA
KSYNRVTVMGGFKVENHTACHCSTCYYHKS

**SEQ ID NO: 2**
NSCELTNITIAIEKEECRFCISINTTWCAGYCYTRDLVYKDPARPKIQKTCTFKELVYETVRVPGC
AHHADSLYTYPVATQCHCGKCDSDSTDCTVRGLGPSYCSFGEMKE

## REFERENCES CITED IN THE DESCRIPTION

### Patent documents cited in the description

- WO 2012017058 A
- US 5272135 A
- US 61263931
- EP 09014585 A
- WO 2011063943 A

### Non-patent literature cited in the description

- **SAMBROOK et al.** Molecular Cloning: A Laboratory Manual. Cold Spring Harbor Laboratory Press, 2001
- **AUSUBEL et al.** Current Protocols in Molecular Biology. J, Greene Publishing Associates, 1992
- Handbook of Biochemistry: Section A Proteins. CRC Press, 1976, vol. I
- Handbook of Biochemistry: Section A Proteins. CRC Press, 1976, vol. II
- *WHO Tech Rep. Series*, 1988 (872)
- **STEELMAN, S.L.** ; **POHLEY, F.M**. *Endocrinology*, 1953, vol. 53, 604-616